# EUROPEAN PATENT APPLICATION

(11) **EP 3 067 369 A1**
(43) Date of publication of application: **14.09.2016**
(21) Application number: 15382110.3
(22) Date of filing: 11.03.2015
(51) Int. Cl.: C07K 16/22, C07K 16/28, C07K 16/30, C07K 16/40, A61K 39/00

(54) **Methods and compositions for the treatment of anti-angiogenic resistant cancer**

(71) Applicant: Institut d'Investigació Biomèdica de Bellvitge (IDIBELL), 08908 Hospitalet de Llobregat (ES); Institut Català d'Oncologia (ICO), 08908 L'Hospitalet de Llobregat (ES)
(72) Inventor: Casanovas Casanovas, Oriol, E-08019 Barcelona (ES); Jiménez Valerio, Gabriela, E- 08028 Barcelona (ES); Ochoa De Olza, María, E-08173 Sant Cugat del Vallès - Barcelona (ES); Martínez Lozano, María de Mar, E-08104 Barcelona (ES)
(74) Representative: ABG Patentes, S.L.

(57) **Abstract**

The present invention corresponds to the field of cancer treatment and is related to compounds and compositions for their use in the treatment of cancer, in particular to inhibitors of platelet derived-endothelial cell growth factor (PD-ECGF) for their use in the treatment of cancer resistant to anti-angiogenic therapy.

## Description

### FIELD OF THE INVENTION

The present invention belongs to the field of cancer treatment and is related to compounds for their use in the treatment of cancer, in particular to PD-ECGF inhibitors for their use in the treatment of cancer that becomes resistant to an anti-angiogenic therapy.

### BACKGROUND OF THE INVENTION

More than 30 years ago, on the basis of the recognition that tumor-associated endothelial cells play a fundamental role in tumor neovascularization, and given their presumed genetic stability, these cells were proposed as a therapeutic target. VEGF-A was identified as a central endothelial cell survival factor and angiogenesis promoter. Bevacizumab (monoclonal antibody against VEGF-A 165) was among the initial antiangiogenic agents developed. This therapeutic strategy has provided clinical benefit in several solid tumor types, and bevacizumab remains approved for colorectal, renal, nonsquamous/non-small-cell lung cancer, and glioblastoma. Although bevacizumab was approved for metastatic breast cancer in 2008, approval was withdrawn secondary to concerns about efficacy relative to toxicity. In ovarian cancer trials, modest improvements in progression-free survival (PFS) were noted in the groups receiving maintenance bevacizumab. Although interval to progression has improved, there seems to have been no improvement in the total number of patients who progressed. Small-molecule inhibitor data are less mature, but similar observations have been made regarding sorafenib and sunitinib, prompting investigation into potential mechanisms of escape from anti-VEGF therapy.

Evolutionary biology teaches that disruptions in an ecosystem by an imposed selection pressure produce reactionary dynamics and interactions, and natural selection will result in a resilient system. Cancer cells are characteristically heterogeneous and genetically unstable. Furthermore, normal cells in the tumor microenvironment such as endothelial cells, pericytes, platelets, fibroblasts, and leukocytes are known to have normal functions that are co-opted to support tumor growth and progression. Potential mechanisms of resistance to antiangiogenic therapy, therefore, may result from the selection of directly and indirectly advantaged subpopulations of tumor and tumor associated cells. If anti-VEGF therapy is considered as a selection pressure, and surviving cell populations are considered to be advantaged in the new environment, multiple plausible mechanisms of resistance and escape emerge for consideration.

Angiogenesis inhibitors targeting the vascular endothelial growth factor (VEGF) signaling pathways are affording demonstrable therapeutic efficacy in mouse models of cancer and in an increasing number of human cancers. However, in both preclinical and clinical settings, the benefits are at best transitory and are followed by a restoration of tumour growth and progression. Emerging data support a proposition that two modes of unconventional resistance underlie such results: evasive resistance, an adaptation to circumvent the specific angiogenic blockade; and intrinsic or pre-existing indifference. Multiple mechanisms can be invoked in different tumor contexts to manifest both evasive and intrinsic resistance.

Bachelor et al. (Cancer Cell 2000, 11:83-95) shows that normalization of tumor vessels in recurrent glioblastoma patients by daily administration of AZD2171 (an oral tyrosine kinase inhibitor of VEGF receptors) has rapid onset, is prolonged but reversible, and has the significant clinical benefit of alleviating edema. This work suggests that fibroblast growth factor bFGF and stromal cell derived factor SDF1α may play a role in glioblastoma relapse in patients treated with anti-VEGF agents.

Fischer et al. (Cell 2007, 131: 463-75) describes antibodies against placental growth factor PIGF (anti-PIGF antibodies) are growth inhibitors of VEGF(R)-inhibitor-resistant tumors.

Kim et al. (Int J Cancer 2013, 132: 29-41) shows that treatment of human sarcomas with bevacizumab, an antibody specific for vascular endothelial growth factor A (VEGF-A) antibody, followed by a combination of bevacizumab and radiation led to near complete necrosis in nearly half of sarcomas. This work suggests that HIF-1α inhibition blocks anti-angiogenic resistance and augments destruction of the tumor vasculature.

van der Bilt et al. (Curr Pharm Des 2012, 18: 3784-92) describes that multiple VEGF family members (VEGF-A, VEGF-B, VEGF-C and VEGF-D) are simultaneously expressed in ovarian cancer, whose expression could contribute to bevacizumab (an anti-angiogenic agent) resistance.

Fan et al. (Br J Cancer 2011, 104: 1270-7) shows that CRC cells exposed to bevacizumab become resistant to the treatment and show increased expression of VEGF-A, -B, -C, P1GF, VEGFR-1 and VEGFR-1 phosphorylation, increased tumor cell migration and invasion, and metastatic potential *in vivo.*

WO2012129448 shows that bevacizumab-resistant glioma cells have β-integrin increased levels and that tumorigenicity of these cells decreases when β-1-integrin is inhibited.

Despite the efforts made to date, there still exists a long-felt and continuing need in the art for novel compounds and/or therapies useful in cancer treatment, particularly in those cancer patients who become irresponsive to anti-angiogenic treatments.

### SUMMARY OF THE INVENTION

The inventors of the present invention have found that PD-ECGF inhibition in *in vivo* tumor models using anti-angiogenic resistant cells results in an increase in the time to progression (TTP) and delays tumor re-growth.

Thus, in a first aspect, the invention relates to a PD-ECGF inhibitor for use in the treatment of cancer in a subject in need thereof, wherein said cancer is resistant to an anti-angiogenic treatment.

In a second aspect, the invention relates to a composition comprising a PD-ECGF inhibitor and an anti-angiogenesis agent.

In a further aspect, the invention relates to the composition of the invention for use in the treatment of cancer, wherein said cancer is resistant to anti-angiogenic treatment.

In a last aspect, the invention relates to an *in vitro* method for determining whether a tumor in a patient is resistant or sensitive to an anti-angiogenesis agent that comprises
(i) determining the expression level of PD-ECGF in a sample from said patient, and
(ii) comparing the expression level in (i) to a reference value,
wherein an increased expression level of PD-ECGF is indicative that the tumor is resistant to anti-angiogenesis agent, and a decreased expression level of PD-ECGF is indicative that the tumor is sensitive to an anti-angiogenesis agent.

### DESCRIPTION OF THE FIGURES

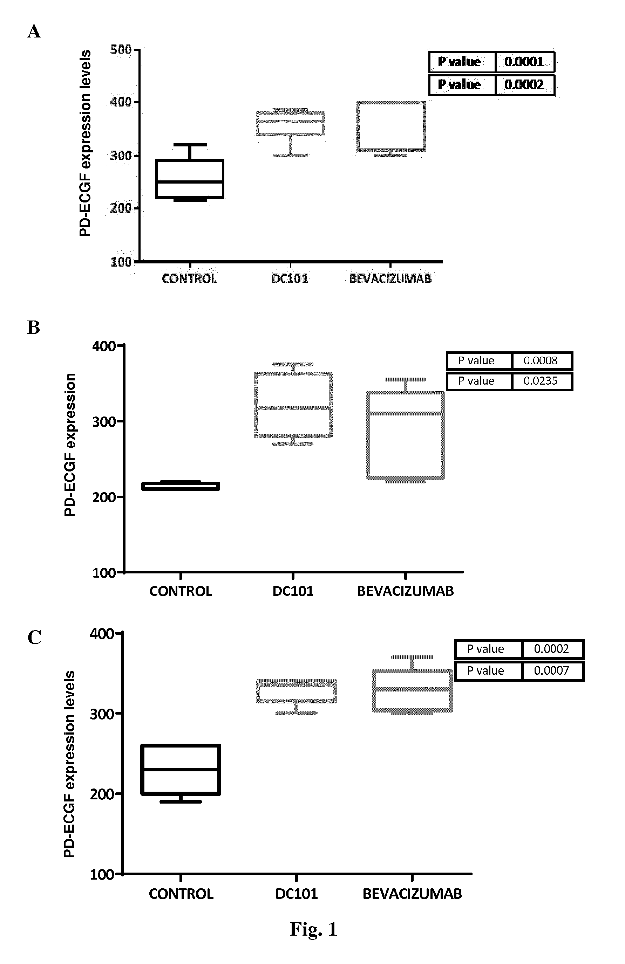

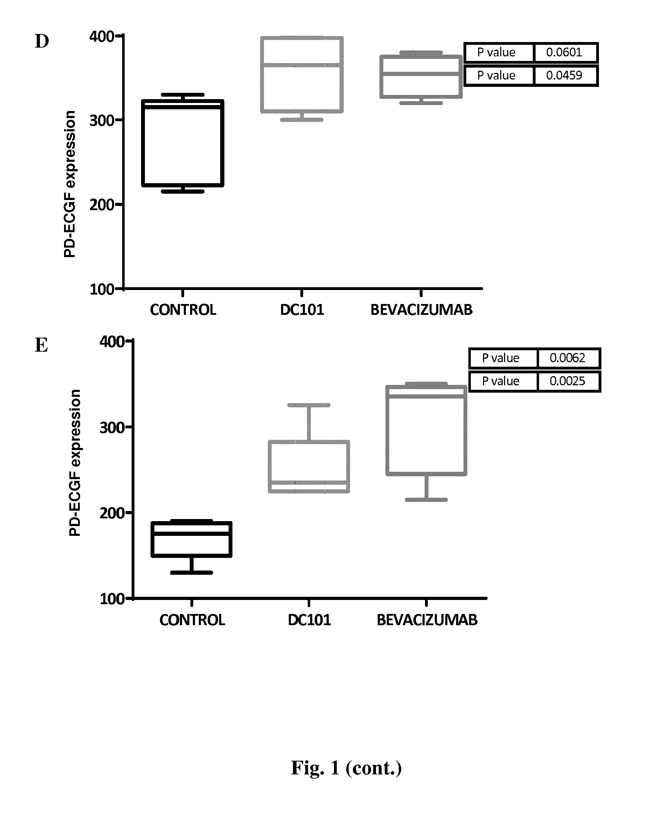

**Figure 1****.** PD-ECGF mRNA expression levels in control tumors and tumors under anti-angiogenic treatment with DC101 (anti-mouse VEGFR2) or bevacizumab (anti-human VEGF). Tumors were derived from 7860- (A), REN 13 (B), REN 28 (C), REN 38 (D) or REN 50 (E) cell lines. Anti-angiogenic treatment was followed for 2 (A), 4 (B), 3 (C), 4.5 (D) or 1 (E) weeks.

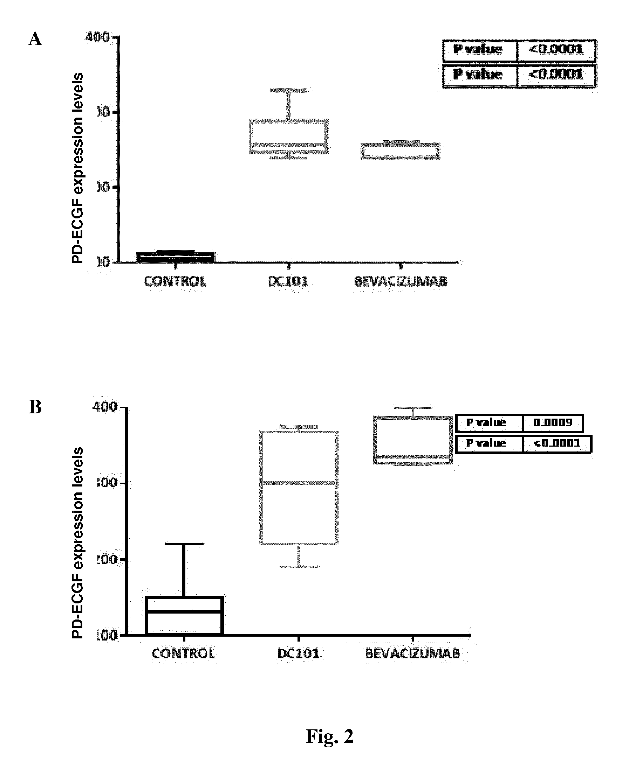

**Figure 2****.** PD-ECGF mRNA expression levels in control tumors and anti-angiogenic resistant tumors under anti-angiogenic treatment with DC101 or bevacizumab. Tumors were derived from 7860- (A) or REN 28 (B) cell lines. Anti-angiogenic treatment was followed for 3 (A) or 4 (B) weeks.

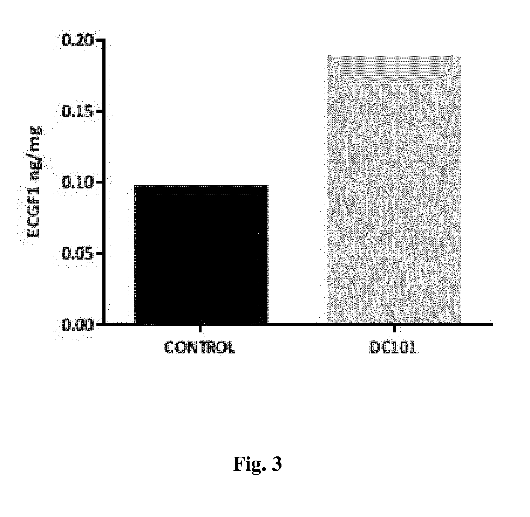

**Figure 3****.** PD-ECGF protein levels determined by ELISA in control tumors and tumors derived from REN 28 under anti-angiogenic treatment with DC101.

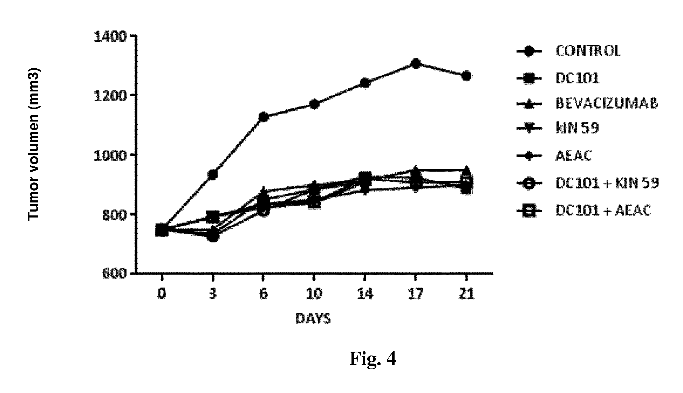

**Figure 4****.** Progression of tumor volume in mice treated with DC101, bevacizumab, KIN59, AEAC, DC101 plus KIN59, and DC101 plus AEAC.

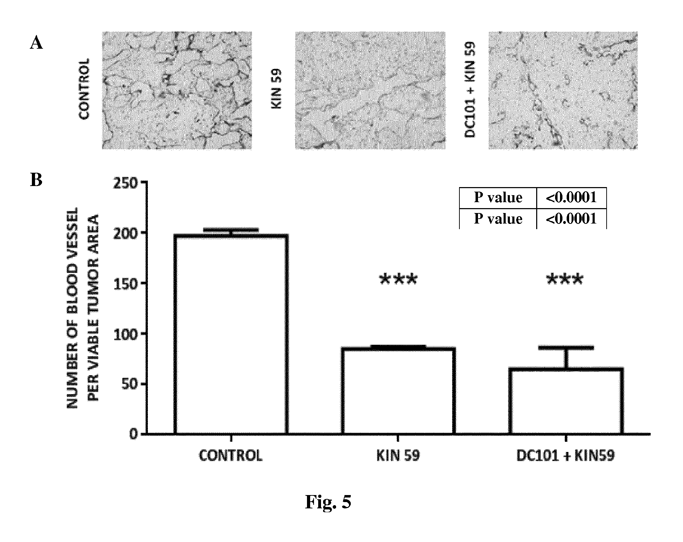

**Figure 5****.** Inhibitory effect on tumor vasculature of KIN 59 (PD-ECGF inhibitor) and the combination of KIN 59 and DC101 (VEGFR2 inhibitor). (A) Immunohistochemistry. A decrease in the number of vessels (labeled with anti-CD31 antibody) is observed after 15 days of treatment. (B) Quantification of vascular density after treatment.

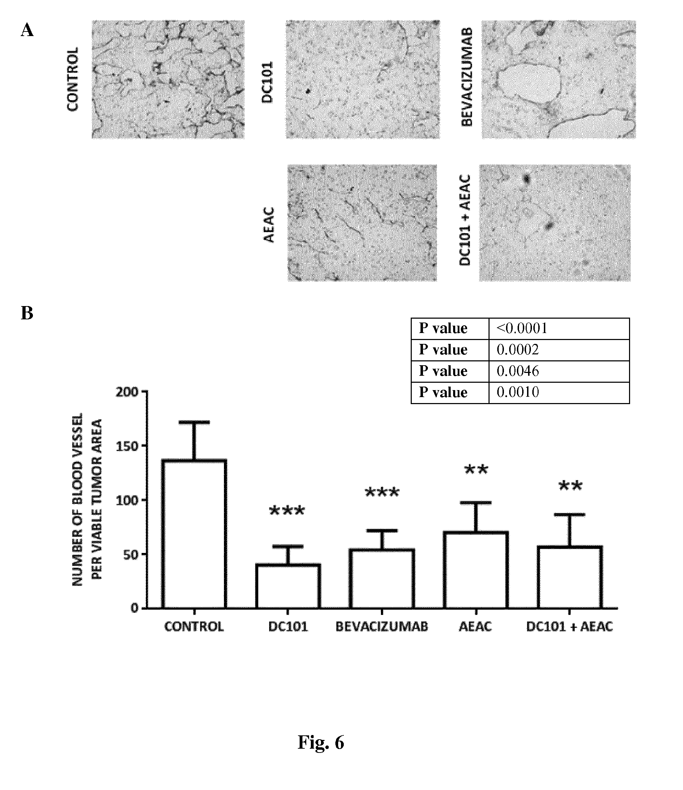

**Figure 6****.** Inhibitory effect on tumor vasculature of bevacizumab (VEGF blocking agent), DC101 (VEGFR2 blocking agent), AEAC (PD-ECGF chemical inhibitor) and the combination of DC101 + AEAC. (A) Immunohistochemistry. A decrease in the number of vessels (labeled with anti-CD31 antibody) is observed after 21 days of treatment. (B) Quantification of vascular density after treatment.

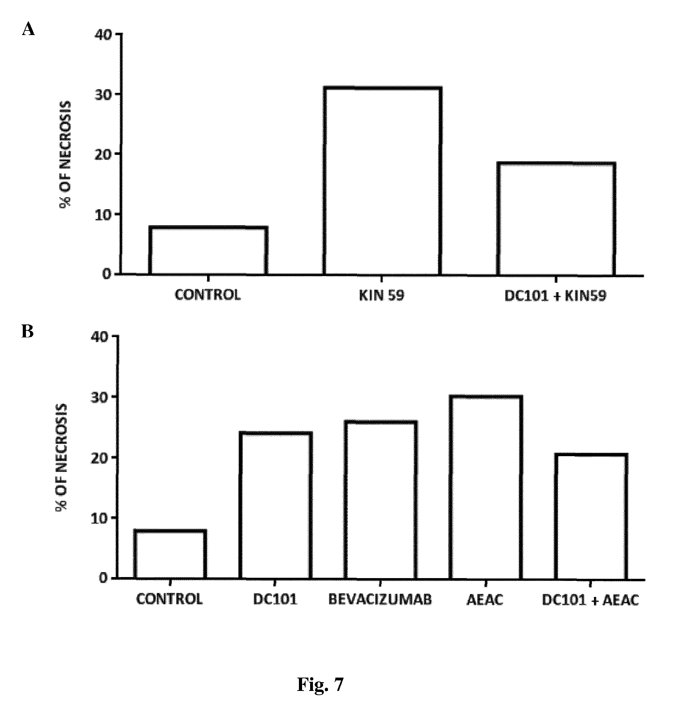

**Figure 7****.** Quantification of cell necrosis after 15 days treatment with KIN 59 or a combination of KIN59 plus DC101 (A) or after 21 days treatment with DC101, bevacizumab, AEAC or a combination of DC101 plus AEAC (B).

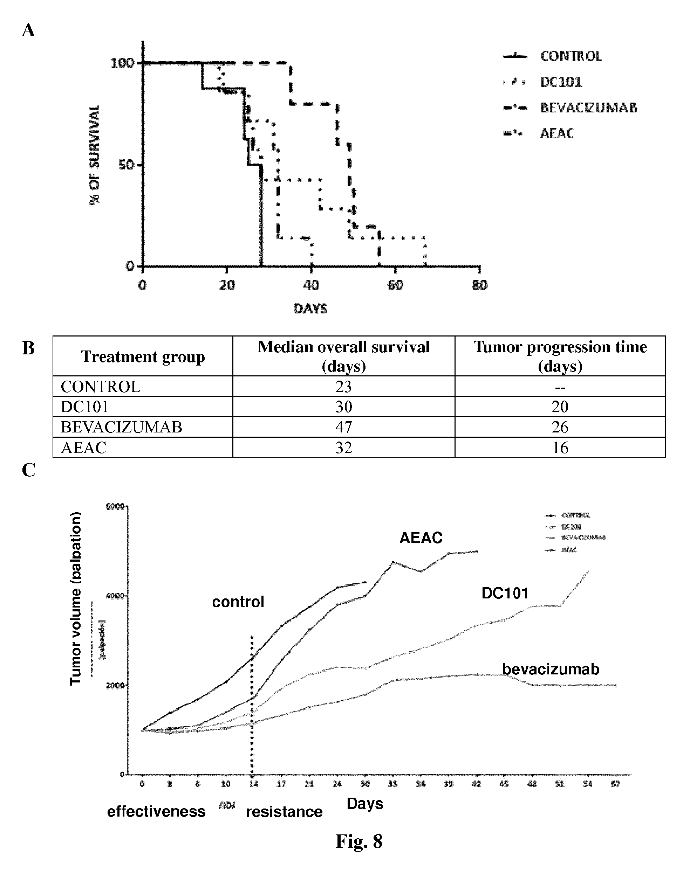

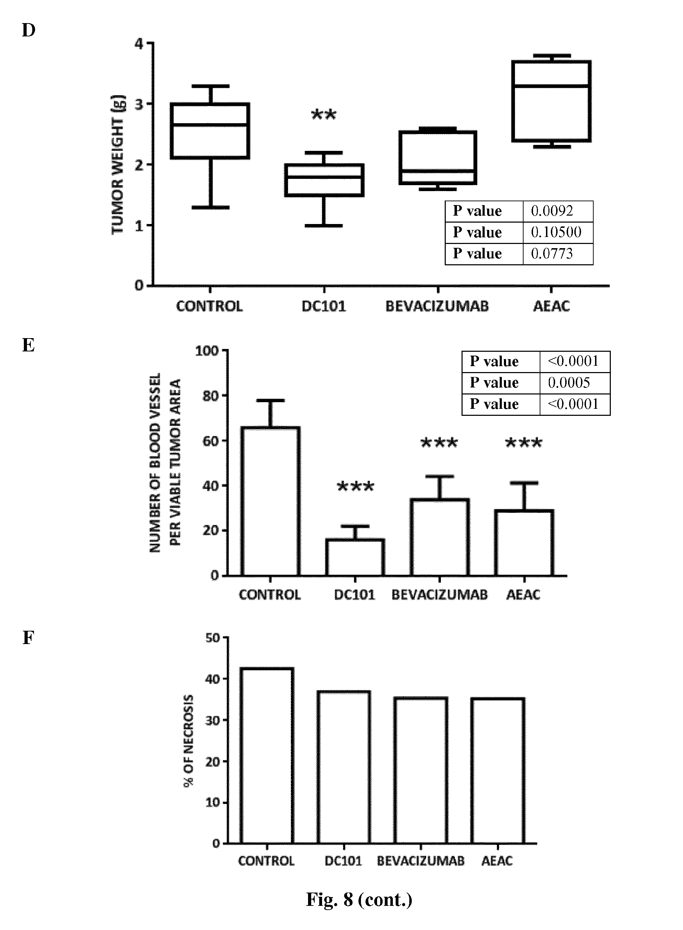

**Figure 8****.** Survival of mice treated with DC101, bevacizumab or AEAC. (A) Kaplan-Meier survival curve of animals with tumors generated from the REN 28 primary tumor. (B) REN 28 survival experiment data. Median overall survival (OS) is the time in days when 50% of the animals are still alive, time to tumor progression (TTP) is the time in days when tumors begin to regrow. (C) Assessment of tumor volume progression (palpation) during treatment with bevacizumab, AEAC and DC101. (D) Effect on tumoral weight in mice under treatment with bevacizumab, AEAC and DC101. (E) Effect on tumor vasculature of bevacizumab, AEAC and DC101. Effect on necrosis of bevacizumab, AEAC and DC 101.

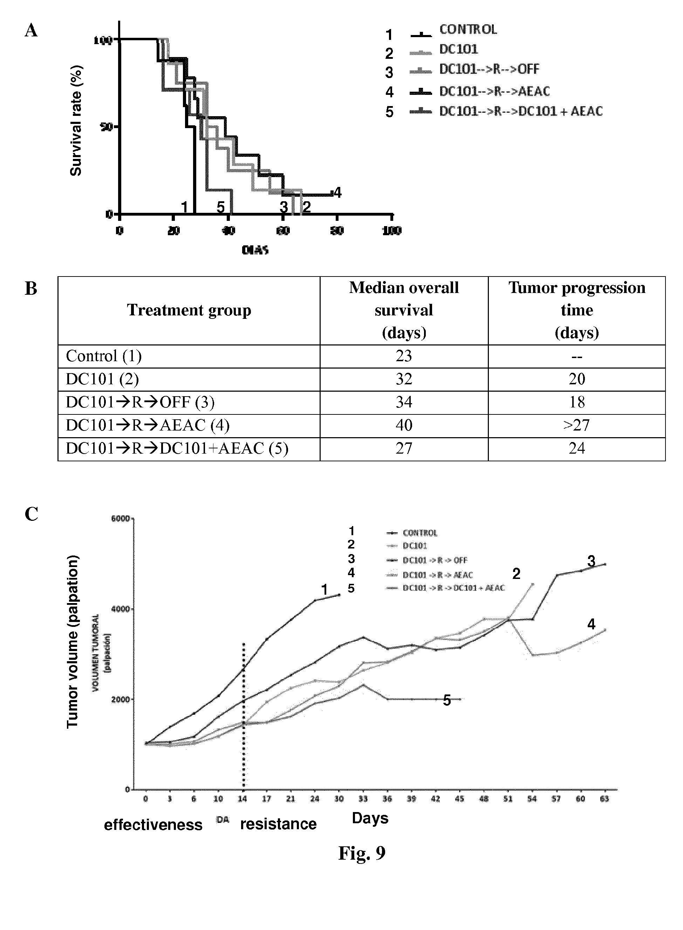

**Figure 9****.** Survival of mice without any treatment after developing anti-angiogenic resistance (DC101→R→OFF), mice under treatment with AEAC after developing anti-angiogenic resistance (DC101→R→AEAC), mice under combination therapy with DC101 and AEAC after developing anti-angiogenic resistance (DC101→R→DC101 + AEAC) and control mice. (A) Kaplan-Meier survival curve of animals with tumors generated from the primary tumor REN 28. (B) REN 28 survival experiment data. Median overall survival (OS) is the time in days when 50% of the animals are still alive, time to tumor progression (TTP) is the time in days when tumors begin to regrow. (C) Assessment of tumor volume progression (palpation).

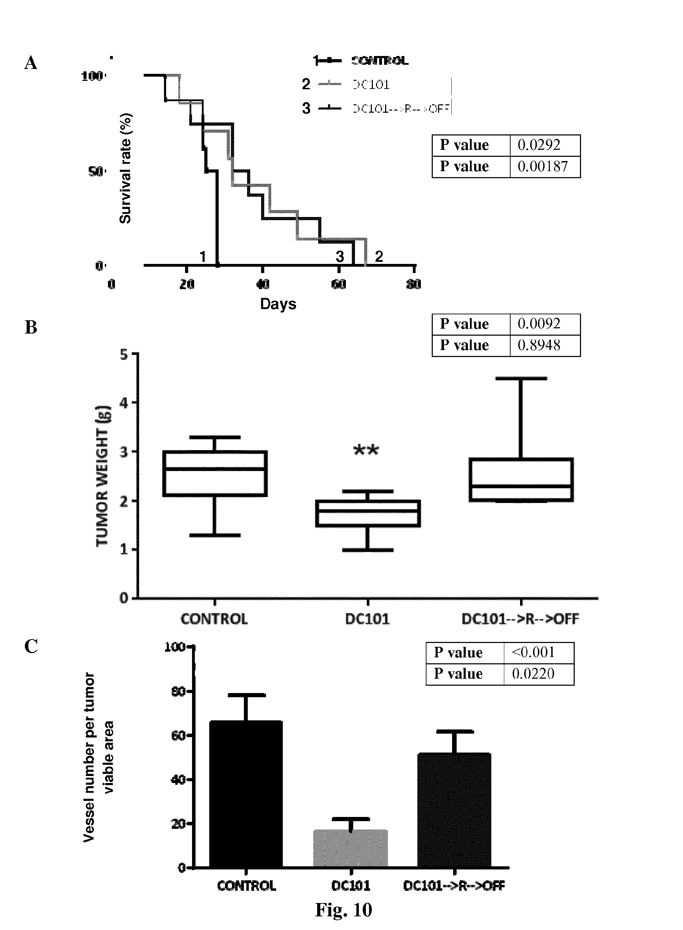

**Figure 10****.** Survival of mice under continuous treatment with DC101 (anti-mouse VEGFR2), mice treated without any treatment after developing anti-angiogenic resistance (DC101→R→OFF) and control mice. DC101 treatment was stopped after a 15-days treatment (start of resistance). (A) Kaplan-Meier survival curve of animals with tumors generated from the primary tumor REN 28. (B) Tumor weight. (C). Tumor vasculature.

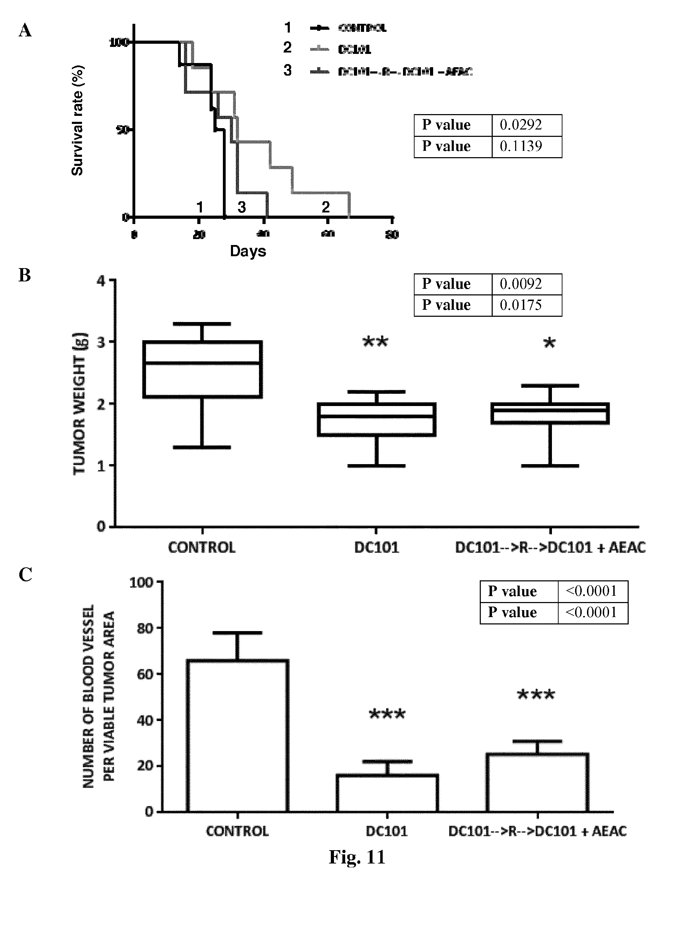

**Figure 11****.** Survival of mice under continuous treatment with DC101 (anti-mouse VEGFR2), mice under treatment with AEAC after developing anti-angiogenic resistance (DC101→R→AEAC) and control mice. (A) Kaplan-Meier survival curve of animals with tumors generated from the primary tumor REN 28. (B) Tumor weight. (C). Tumor vasculature.

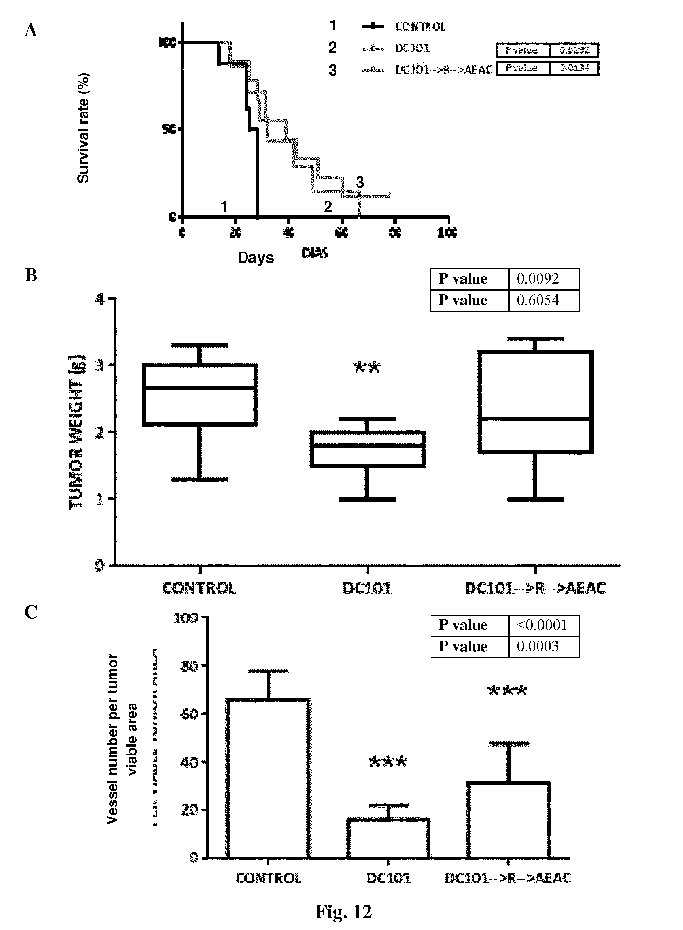

**Figure 12****.** Survival of mice under continuous treatment with DC101 (anti-mouse VEGFR2), mice under combination therapy with DC101 and AEAC after developing anti-angiogenic resistance (DC101→R→DC101 + AEAC) and control mice. (A) Kaplan-Meier survival curve of animals with tumors generated from the primary tumor REN 28. (B) Tumor weight. (C). Tumor vasculature.

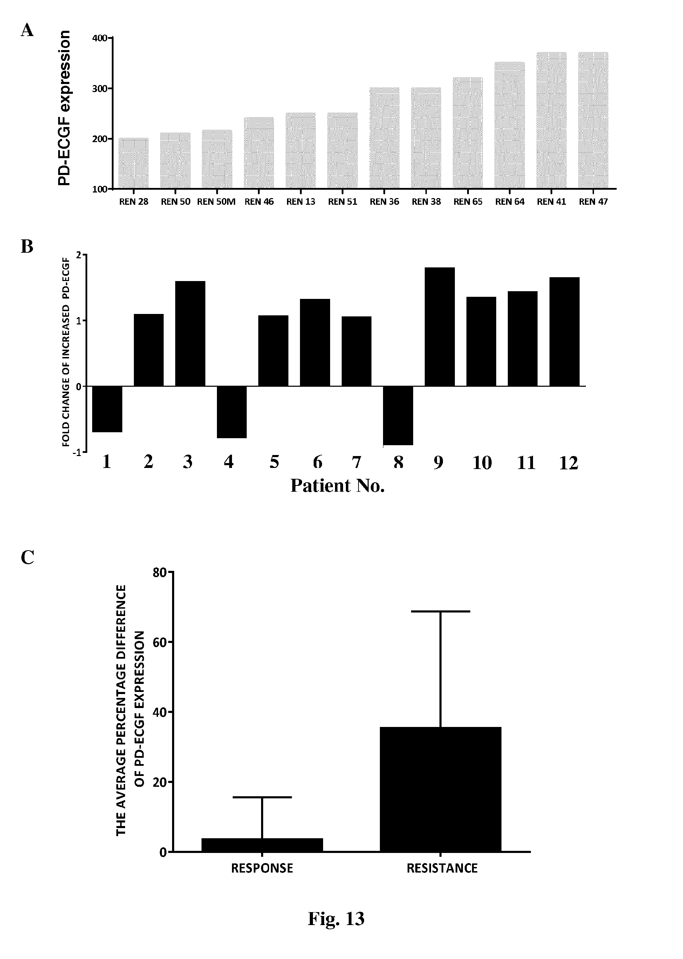

**Figure 13****.** PD-ECGF analysis in tumor samples from RCC (renal cell carcinoma) cancer patients. (A) PD-ECGF expression level in orthotopic tumors generated from patients biopsies. (B) PD-ECGF expression level in tumors samples from RCC cancer patients obtained before and after the anti-angiogenic treatment. (C) PD-ECGF expression percentage difference in samples from patients according to their progression.

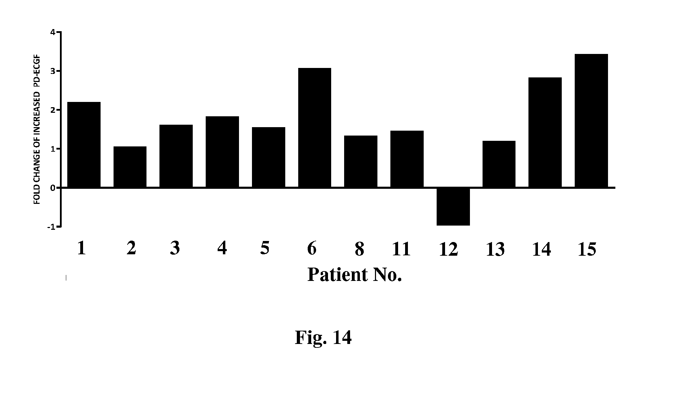

**Figure 14****.** PD-ECGF analysis in tumor samples from breast cancer patients. (A) PD-ECGF expression level in tumors samples from breast cancer patients obtained before and after the anti-angiogenic treatment.

### DETAILED DESCRIPTION OF THE INVENTION

The authors of the present invention have found that PD-ECGF (also known as ECGF1) expression increases in tumor cells under anti-angiogenic treatment with DC101 and bevacizumab (see Example 1), and that this increased expression continues when the tumor cell becomes resistant to the anti-angiogenic treatment (see Example 2). The authors have assayed ECGF1 inhibitors, particularly KIN59 and AEAC, in tumor cells and demonstrated that there is a comparable effectiveness between PD-ECGF inhibitors and anti-angiogenic therapies (bevacizumab and DC101), and that the inhibition of the PD-ECGF pathway results in decreased weight and tumor volume, decreased tumor vasculature and increased tumor necrosis (see Example 3). Additionally, they have demonstrated that inhibition of the PD-ECGF pathway is also effective in the treatment of tumors resistant to an anti-angiogenic therapy (see Example 3). Finally, the inventors have determined that the expression level of PD-ECGF in a tumor sample from a subject may be used as an indicator of the response of said tumor to an anti-angiogenic agent (Example 4).

### Definitions

The term "AEAC", as used herein, relates to 6-(2-aminoethyl)amino-5-chlorouracil, according to the following formula, and which is an inhibitor of PD-ECGF.

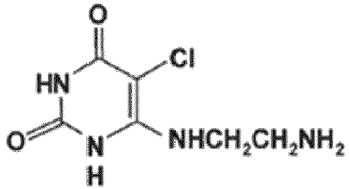

The term "anti-angiogenic agent" or "anti-angiogenesis agent" or "angiogenesis inhibitor", as used in the present invention, relates to an agent targeted to angiogenesis (e.g. the process of forming blood vessels) including, but not limited to, tumor angiogenesis. In this context, inhibition can refer to blocking the formation of blood vessels and halting or slowing down the growth of blood vessels.

The term "anti-angiogenic treatment" or "anti-angiogenesis treatment", as used herein, relates to a treatment based on at least one anti-angiogenesis agent.

The term "bevacizumab", also known as Avastin®, relates to a recombinant humanized monoclonal IgG1 antibody that specifically binds and blocks the biological effects of VEGF. Thus, bevacizumab is considered as a blocking agent of VEGF. The term "VEGF" or "vascular endothelial growth factor", as used herein, relates to a signal protein produced by cells that stimulates vasculogenesis and angiogenesis.

The term "cancer" is referred to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis. The term cancer includes, without limitation, lung cancer, sarcoma, malignant melanoma, pleural mesothelioma, bladder carcinoma, prostate cancer, pancreas carcinoma, gastric carcinoma, ovarian cancer, hepatoma, breast cancer, colorectal cancer, kidney cancer, esophageal cancer, suprarenal cancer, parotid gland cancer, head and neck carcinoma, cervix cancer, endometrial cancer, liver cancer, mesothelioma, multiple myeloma, leukaemia, and lymphoma. In a particular embodiment of the invention, the cancer is any cancer showing intrinsic or acquired resistance to an anti-angiogenic therapy, including kidney cancer, and breast cancer. The term "kidney cancer" relates to cancer of the tissues of the kidneys and includes, without limitation, renal cell carcinoma, renal pelvis carcinoma and Wilms tumor. The term "breast cancer" relates to any malignant proliferative disorder of breast cells, most commonly from the inner lining of milk ducts or the lobules that supply the ducts with milk. Cancers originating from ducts are known as ductal carcinomas, while those originating from lobules are known as lobular carcinomas.

The term "composition", as used herein, relates to a material composition that comprises at least two components, as well as any product resulting, directly or indirectly, from the combination of the different components in any quantity thereof. Those skilled in the art will observe that the composition may be formulated as a single formulation or may be presented as separate formulations of each of the components, which may be combined for joint use as a combined preparation. The composition may be a kit-of-parts wherein each of the components is individually formulated and packaged.

The term "DC101", as used herein, relates to a mouse monoclonal antibody that functions as a blocking agent of vascular endothelial growth factor receptor 2 (VEGFR2, also known as KDR/Flk-1).

The term "expression level", as used herein, refers to the measurable quantity of gene product produced by the gene in a sample of the subject, wherein the gene product can be a transcriptional product or a translational product. As understood by the person skilled in the art, the gene expression level can be quantified by measuring the messenger RNA levels of said gene or of the protein encoded by said gene. In the context of the present invention, the expression level of the gene encoding PD-ECGF can be determined by measuring the levels of mRNA encoded by said gene, or by measuring the levels of the protein encoded by said gene, i.e. PD-ECGF protein or of variants thereof. PD-ECGF protein variants include all the physiologically relevant post-translational chemical modifications forms of the protein, for example, glycosylation, phosphorylation, acetylation, etc., provided that the functionality of the protein is maintained. Said term encompasses the PD-ECGF protein of any mammal species, including but not being limited to domestic and farm animals (cows, horses, pigs, sheep, goats, dogs, cats or rodents), primates and humans. Preferably, the PD-ECGF protein is a human protein.

The term "KIN59", as used herein, relates to an allosteric inhibitor of PD-ECGF according to the formula:

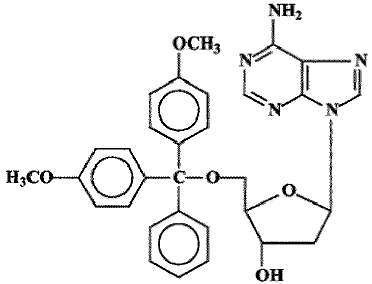

The term "plateled derived-endothelial cell growth factor" or "PD-ECGF", as used herein, is also known as "plateled-derived endothelial cell growth factor-1", "ECGF-1" or "ECGF1", "gliostatin", "platelet-derived endothelial cell mitogen" "thymidine phosphorylase" or "TP", and relates to a cytoplasmic protein initially isolated from platelets showing endothelial mitogenic activity. It is an acidic non-glycosylated protein of 45 kDa, which is synthesized as a precursor of 482 amino acids from which it is derived by N-terminal processing. The protein isolated from placenta contains five additional amino acids at the N-terminus. PD-ECGF can be phosphorylated *in vivo* at serine residues but the biological significance of this phosphorylation step is unknown. The human gene encoding PD-ECGF is located on chromosome 22 and assigned Gene ID 1890 (NCBI GenBank, 1 march 2014 update). The term PD-ECGF includes any of the transcript variants that have been described for this polypeptide, including, transcript variant 1 (accession number NM_001113755.2 in NCBI GenBank), encoding the same isoform 1 than variants 2, 3 and 4; transcript variant 2 (NM_001953.4), which uses an alternate splice site in the 5' UTR; transcript variant 3 (NM_001113756.2), which differs in the 5' UTR compared to variant 1; transcript variant 4 (NM_001257988.1), which uses an alternate splice site in the 5' UTR compared to variant 1; and transcript variant 5 (NM_001257989.1), which uses alternate splice sites in the 5' UTR and the 3' coding region compared to variant 1, and coding for isoform 2, said isoform 2 having an additional segment in the C-terminal region compared to isoform 1. The amino acid sequence of human PD-ECGF is located in NCBI GenBank under accession number AAB03344.2 (482 amino acids, version as of 3 february 2000) and under UniProtKB/Swiss-Prot accession number P19971.2 (Uniprot version 167 as of 19 february 2014).

The term "PD-ECGF inhibitor", as used herein, also known as PD-ECGF inhibitor, relates to a compound inhibiting PD-ECGF gene expression and/or PD-ECGF enzymatic activity.

The term "reference value", as used herein, refers to a laboratory value used as a reference for values/data obtained by laboratory examinations of subjects or samples collected from subjects. The reference value or reference level can be an absolute value, a relative value, a value that has an upper or a lower limit, a range of values, an average value, a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. Suitable reference values are indicated in the context of the method of the invention for determining tumor resistance or sensitivity to an anti-angiogenic treatment.

The term "resistance" as used in the present invention, relates to the resistance developed to a treatment, in particular the resistance developed to an anti-tumoral agent, more particularly the resistance developed to an anti-angiogenic agent. In general, systemic agents are active at the beginning of therapy in cancers and/or metastases, which is demonstrated by reduced tumor volume, improved symptoms, and decreased serological tumor markers. However, after a variable period, progression occurs. Multiple mechanisms of both *in vitro* and *in vivo* resistance have been identified, including cellular and biochemical mechanisms (decreased drug accumulation by decreased drug influx, increased drug efflux of altered intracellular drug trafficking; increased inactivation of drug or toxic intermediate; increased repair of or tolerance to drug-induced damage to DNA, protein or membranes; decreased drug activation; altered drug targets; altered co-factor or metabolite levels; altered downstream effectors of cytotoxicity; altered signaling pathway and/or apoptotic responses to drug insult, due to altered gene expression, DNA mutation, amplification, or deletion, altered transcription, posttranscriptional processing, or translation or altered stability of macromolecules) or by *in vivo* mechanisms (pharmacological and anatomic drug barriers; and host-drug interactions such as increased drug inactivation by normal tissues, decreased drug activation by normal tissues or relatively increased normal tissue drug sensitivity). It has been proposed that resistance to therapy is caused in part by a process called genetic amplification. This process allows cancer cells to increase their immortality and invasion properties. Each treatment regimen with a single systemic agent selects a group of cancer cells that is increasingly resistant to therapy, decreasing the rate of response to further therapies.

Resistance to a therapy may develop as *de novo* resistance or as acquired resistance. The term *"**de novo* resistance" or "intrinsic resistance" or "primary resistance" relates to resistance present before drug exposure and selection for drug resistance, as a failure to respond to initial drug therapy. The term "acquired resistance" or "secondary resistance" relates to resistance occurring following drug selection, wherein an initial response to drug therapy is followed by subsequence disease progression. In particular, the term "anti-angiogenic therapy/agent-resistance" relates to a subject receiving an anti-angiogenic agent-based therapy and who lacks demonstration of a desired physiological effect, such as a therapeutic benefit, from the administration of the therapy, due to *de novo* therapy-resistance or to acquired therapy-resistance.

The term "sensitivity", as used in the present invention, relates to the sensitivity or responsiveness to a treatment, in particular the sensitivity of a tumor cell to an anti-tumoral agent, more particularly the sensitivity of a tumor cell to an anti-angiogenic agent. The term "subject" or "individual" or "animal" or "patient" or "mammal," relates to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age, sex or race.

The term "sunitinib", also known as Sutent® (Pfizer), relates to a multikinase inhibitor, approved by the US Food and Drug Administration for the treatment of gastrointestinal stromal tumors after failure of imatinib mesylate due to disease progression or drug intolerance and for advanced renal cell carcinoma. Sunitinib inhibits receptor tyrosine kinases involved in cell signaling, including vascular endothelial growth factor receptors (VEGFR) 1-3, platelet-derived growth factor receptors (PDGFR) α and β, stem cell factor receptor (KIT), Fms-like tyrosine kinase 3, colony-stimulating factor receptor type 1, and the glial-cell-line-derived neurotrophic factor receptor (RET), all of which have been implicated in tumor growth, angiogenesis and metastatic progression.

The term "therapy", as used herein, refers to the attempted remediation of a health problem, usually following a diagnosis, or to prevention or the appearance of a health problem. As such, it is not necessarily a cure, i.e. a complete reversion of a disease. Said therapy may or may not be known to have a positive effect on a disease, such as cancer. This term includes both therapeutic treatment and prophylactic or preventative measures, in which the object is to prevent or stop (reduce) an undesired physiological change or disorder. For the purpose of this invention, beneficial or desired clinical results include, without limitation, relieving symptoms, reducing the spread of the disease, stabilizing pathological state (specifically not worsening), slowing down or stopping the progression of the disease, improving or mitigating the pathological state and remission (both partial and complete), both detectable and undetectable. It can also involve prolonging survival in comparison with the expected survival if treatment is not received. Those subjects needing treatment include those subjects already suffering the condition or disorder, as well as those with the tendency to suffer the condition or disorder or those in which the condition or disorder must be prevented.

The term "treatment", as used herein comprises any type of therapy, which aims at terminating, preventing, ameliorating and/or reducing the susceptibility to a clinical condition as described herein. In a preferred embodiment, the term treatment relates to prophylactic treatment (i.e. a therapy to reduce the susceptibility of a clinical condition, a disorder or condition as defined herein). Thus, "treatment", "treating", and the like, as used herein, refer to obtaining a desired pharmacologic and/or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, and/or immune deficiency.

### PD-ECGF inhibitors for use in the treatment of anti-angiogenic treatment-resistant cancer

The authors of the present invention have found that inhibition of the PD-ECGF pathway reduced tumor volume and increased time to progression (TTP) in anti-angiogenic treatment-resistant tumors (see Examples 3 and 4).

Thus, in a first aspect, the invention relates to a PD-ECGF inhibitor for use in the treatment of cancer in a subject in need thereof, wherein said cancer is resistant to an anti-angiogenic treatment. Alternatively, the invention relates to a PD-ECGF inhibitor for the manufacture of a medicament for the treatment of cancer, wherein said cancer is resistant to an anti-angiogenic treatment. Alternatively, the invention relates to a method for the treatment of cancer in a subject in need thereof that comprises the administration of a therapeutically effective amount of a PD-ECGF inhibitor according to the invention to a subject suffering from cancer, wherein said cancer is resistant to an anti-angiogenic treatment.

According to the invention, PD-ECGF inhibitors comprise any compound capable of causing a decrease in the PD-ECGF activity (including those compounds which prevent expression of the PD-ECGF gene, leading to reduced PD-ECGF mRNA or protein levels, decreasing the expression levels of the gene), as well as compounds that inhibit PD-ECGF causing a decrease in the thymidine phosphorylase activity.

Suitable methods for determining whether an inhibitor is capable of decreasing the PD-ECGF mRNA levels include, without limitation, standard assays for determining mRNA expression levels such as qPCR, RT-PCR, RNA protection analysis, Northern blot, RNA dot blot, *in situ* hybridization, microarray technology, tag based methods such as serial analysis of gene expression (SAGE) including variants such as LongSAGE and SuperSAGE, microarrays, fluorescence in situ hybridization (FISH), including variants such as Flow-FISH, qFiSH and double fusion FISH (D-FISH), and the like.

Thus, the gene expression level can be quantified by measuring the messenger RNA levels of the PD-ECGF encoding gene or of the protein encoded by said gene. In the context of the present invention, the expression level of the PD-ECGF encoding gene can be determined by measuring the levels of mRNA encoded by said gene, or by measuring the levels of the protein encoded by said gene, i.e. PD-ECGF protein or of variants thereof. PD-ECGF protein variants include all the physiologically relevant post-translational chemical modifications forms of the protein, for example, glycosylation, phosphorylation, acetylation, etc., provided that the functionality of the protein is maintained. Said term encompasses the PD-ECGF protein of any mammal species, including but not being limited to domestic and farm animals (cows, horses, pigs, sheep, goats, dogs, cats or rodents), primates and humans. Preferably, the PD-ECGF protein is a human protein.

Suitable methods for determining whether an inhibitor acts by decreasing the PD-ECGF protein levels include, without limitation, Western-blot or Western transfer, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of protein biochips or microarrays which include specific antibodies or assays based on colloidal precipitation in formats such as dipsticks.

Suitable methods for determining whether an inhibitor acts by decreasing the PD-ECGF activity include any method which allows determining the thymidine phosphorylase activity. Assays to determine the activity level of an enzyme are known by the skilled person and include, without limitation, initial rate assays, progress curve assays, transient kinetics assays and relaxation assays. Continuous assays of enzymatic activity include, without limitation, spectrophotometric, fluorometric, calorimetric, chemiluminiscent, light scattering and microscale thermopheresis assays. Discontinuous assays of enzymatic activity include, without limitation, radiometric and chromatographic assays. As the skilled person understands, factors that may influence enzymatic activity comprise salt concentration, temperature, pH, and substrate concentration.

The determination of the inhibitory capacity on the biological activity of PD-ECGF is detected using standard assays to measure the thymidine phosphorylase activity of PD-ECGF such as the methods based on the detection of the phosphorylisis of thymidine, as described in Miyadera K et al. (Cancer Res., 1995, 55: 1687-1690) or be measuring PD-ECGF growth-promoting activity by determining the incorporation of ³H-thymidine into the DNA of endothelial cells, as described in US 5227302 A.

Exemplary PD-ECGF inhibitors for use according to the invention in the treatment of anti-angiogenic treatment resistant-cancer are shown in Table 1.

**Table 1. PD-ECGF inhibitors for use according to the invention**

| | | | |
|---|---|---|---|
| **I** | Thymidine phosphorilase inhibitors according to WO2005/026131 (Figure 2), based on transition-state structure of thymidine phosphorylase, as follows: | | |
| | In all cases, R³ = halogen (preferably Cl), H, or lower alkyl (preferably Me) | | |
| | 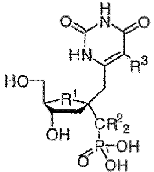 | 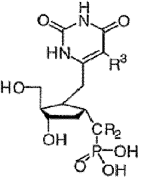 | 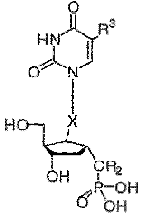 |
| | **1** R¹=O R²=F | **5** R=F | **7** R=F, X=CH₂ or nothing |
| | **2** R¹=O R²=H | **6** R=H | **8** R=H, X=CH₂ or nothing |
| | **3** R¹=CH₂ R²=F | 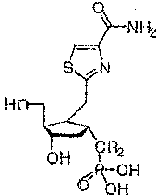 | 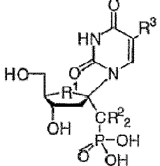 |
| | **4** R¹=CH₂ R²=H | | |
| | 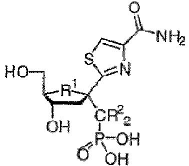 | | |
| | | | **9** R¹=O R²=F |
| | | **13** R=F | **10** R¹=O, R²=H |
| | **15** R¹=O, R²=F | **14** R=H | **11** R¹=CH₂, R²=F |
| | **16** R¹=O, R²=H | | **12** R¹=CH₂, R²=H |
| | **17** R¹=CH₂, R²=F | 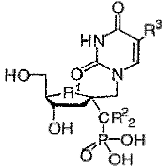 | |
| | **18** R¹=CH₂, R²=H | | |
| | | **19** R¹=O, R²=F | |
| | | **20** R¹=O, R²=H | |
| | | **21** R¹=CH₂, R²=F | |
| | | **22** R¹=CH₂, R²=H | |
| **II** | Thymine phosphonomethoxyalkyl derivatives as described in Votruba I *et al.* | | |
| | 2005 Biochem Pharmacol 69(10): 1517-1521, including the acyclic nucleotide analogues (R)-FPMPT, (S)-FPMPT, (R)-HPMPT, (S)-PMPT, (S)-HPMPT, PMET and (R)-PMPT. | | |
| **III** | Allosteric inhibitors of PD-ECGF including, without limitation, TP65, KIN56, KIN59 ( Liekens S et al. 2004 J Biol Chem 279: 29598-29605), and derivatives thereof (Liekens S et al. 2006 Mol Pharmacol 71: 501-509). | | |
| | TP65 is a compound based on (1-(8-phosphonooctyl)-7-deazaxanthine according to the following formula | | |
| | 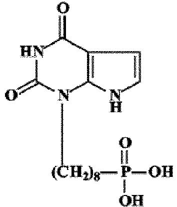 | | |
| | KIN56 is a compound based on 5'-*O*-(4,4'-dimethoxytrityl)-2'-deoxyadenosine according to the following formula | | |
| | 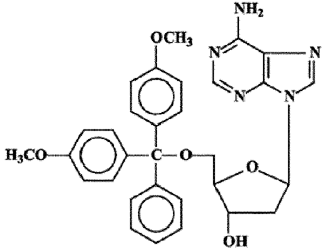 | | |
| | KIN59 is a compound based on 5'-O-trityl-inosine according to the formula | | |
| | 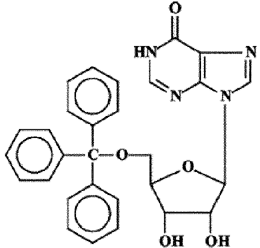 | | |
| | KIN59 derivatives include, without limitation, the compounds TP142, TP146, | | |
| | TP136, TR140, TP124, KIN6, TP147, TP134, TP137, D7154, TA-01, TP141, TP151, as described in Liekens S et al. 2006 Mol Pharmacol 70: 501-509, as follows: | | |
| | 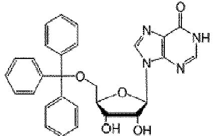 | 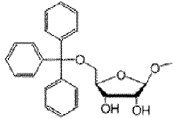 | |
| | KIN59 | TP142 | |
| | 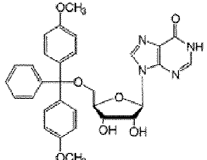 | 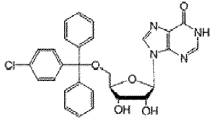 | 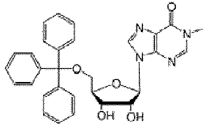 |
| | TP146 | TP136 | TP140 |
| | 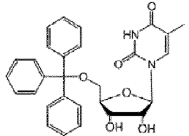 | 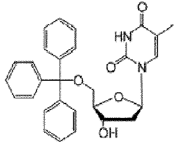 | 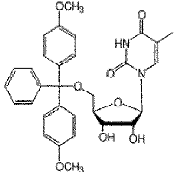 |
| | TP124 | KIN6 | TP147 |
| | 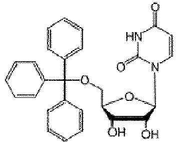 | 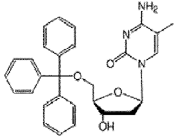 | 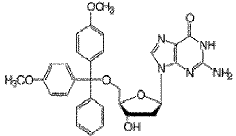 |
| | TP134 | TP137 | D7154 |
| | 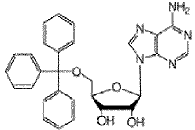 | 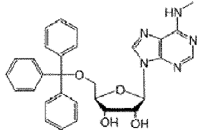 | 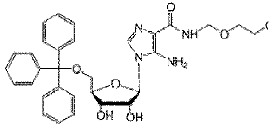 |
| | TA-01 | TP141 | TP151 |
| **IV** | 6-(2-aminoethyl)amino-5-chlorouracil (AEAC) according to the following formula: | | |
| | 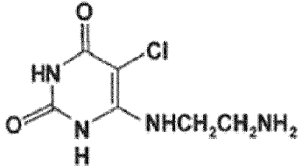 | | |
| **V** | TP inhibitors as described by Bronckaers (Bronckaers A et al. 2009 Med Res Rev 29(6): 903-953) as follows: | | |
| | 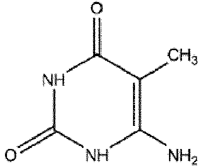 | 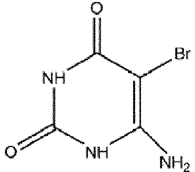 | |
| | **6AT** | **6A5BU** | |
| | 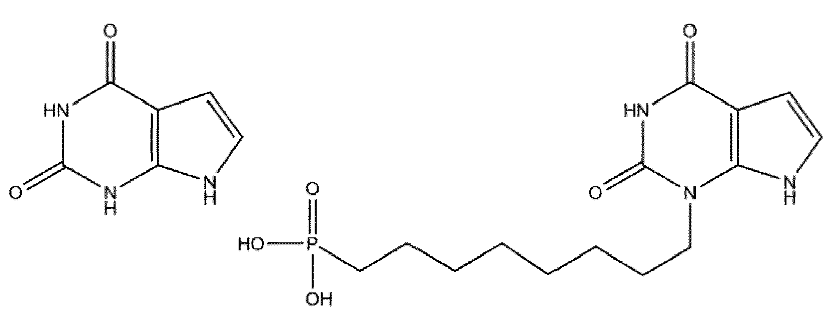 | | |
| | **7DX** | **TP65** | |
| | 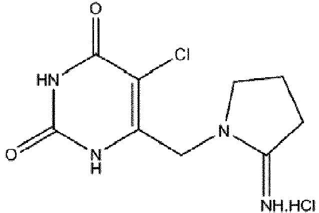 | 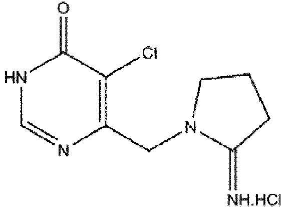 | |
| | **TPI** | **XO-directed prodrug of TPI** | |

In a particular embodiment, the PD-ECGF inhibitor for use according to the invention is selected from the group comprising an anti-PD-ECGF antibody, a PD-ECGF specific RNA interference (RNAi), a PD-ECGF specific ribozyme, a PD-ECGF specific antisense nucleic acid, a PD-ECGF specific DNA enzyme, and a PD-ECGF inhibitor according to Table 1.

In a particular embodiment, the PD-ECGF inhibitor for use according to the invention is selected from the inhibitors as shown in Table 1. In a more particular embodiment, the PD-ECGF inhibitor for use according to the invention is selected from the group comprising TP65, KIN56, a derivative thereof including those compounds as described in Liekens S et al. 2006 Mol Pharmacol 70: 501-509, AEAC, and an shRNA. In a more particular embodiment, the PD-ECGF inhibitor is selected from the group comprising KIN59 and AEAC.

In a particular embodiment, the PD-ECGF inhibitor for use according to the invention is an anti-PD-ECGF specific inhibitory antibody. Antibodies against an epitope located PD-ECGF may effectively block the function of this protein. "Inhibitory antibody", as used herein, refers to antibodies which are capable of inhibiting at least partially the biological activity of PD-ECGF.

The determination of the inhibitory capacity on the biological activity of PD-ECGF is detected using assays to measure the activity of PD-ECGF as described in Miyadera K et al. 1995 and US 5227302 A (*ad supra*).

Thus, the inhibitory antibody for use in the invention is capable of binding an epitope of PD-ECGF; typically, at least 6, 8, 10, or 12, contiguous amino acids are required to form an epitope, however, epitopes which involve non-contiguous amino acids may require more, e.g., at least 15, 25, or 50 amino acid. The term "antibody" includes, for example, polyclonal antibodies, monoclonal antibodies, Fab and single chain Fv (scFv) fragments thereof, bispecific antibodies, heteroconjugates, human and humanized antibodies. Such antibodies may be produced in a variety of ways, including hybridoma cultures, recombinant expression in bacteria or mammalian cell cultures, and recombinant expression in transgenic animals. Also antibodies can be produced by selecting a sequence from a library of sequences expressed in display systems such as filamentous phage, bacterial, yeast or ribosome. There is abundant guidance in the literature for selecting a particular production methodology, e.g., Chadd and Chamow, Curr. Opin. Biotechnol., 12:188-194 (2001). The choice of manufacturing methodology depends on several factors including the antibody structure desired, the importance of carbohydrate moieties on the antibodies, ease of culturing and purification, and cost. Many different antibody structures may be generated using standard expression technology, including full-length antibodies, antibody fragments, such as Fab and Fv fragments, as well as chimeric antibodies comprising components from different species. Antibody fragments of small size, such as Fab and Fv fragments, having no effector functions and limited pharmokinetic activity may be generated in a bacterial expression system. Single chain Fv fragments show low immunogenicity and are cleared rapidly from the blood.

The antibodies may also be engineered to alter its clinical uses. Numerous approaches make use of the molecular biology and genetic techniques such as the good knowledge of the genetics ad structure of the immunoglobulins to construct different modifications of immunoglobulin molecule with the aim of improve its properties for clinical or other uses. Some of them tend to reduce the immunogenicity of the molecule in the species in which should be used and the resultant molecule has a sequence more homologous with this species. Various methods have been used to obtain mAbs of human origin avoiding the non-ethically admissible proceedings in healthy humans. In other approaches the molecular weight and size are reduced e.g. in order of improving the distribution of the molecule into solid tumours. Other possibilities are conjugation in a molecule of binding domains for more than one target molecule (bispecific antibody or also triespecific, etc) or the conjugation of an antibody or a fragment with another molecule with the desired function e.g. a toxic agent, a hormone, growth factor, a immunomodulating agent (immunosuppressor or immunostimulator), an inhibitor of cell growth, etc. In general all the resultant molecules retain almost one variable domain of an antibody which gives the high specificity and affinity characteristic of the antigen-antibody binding. Some examples of these constructions are chimeric antibodies, humanized antibodies, primatized antibodies, human antibodies, antibody fragments such as Fab, F(ab')2, Fab' and scFv, diabodies, bispecific antibodies.

Exemplary anti-PD-ECGF antibodies according to the invention include, without limitation, commercial antibodies including the anti-human PD-ECGF neutralizing antibody for recombinant human PD-ECGF (R&D Systems, Catalog No. AB-229-NA), and the polyclonal anti-human ECGF1 antibody from Cell Signaling Technology (Catalog No. 2937).

In a particular embodiment, the PD-ECGF inhibitor for use according to the invention is a PD-ECGF specific RNA interference (RNAi), i.e. RNAi which are capable of knocking down the expression of PD-ECGF or any component gene necessary for PD-ECGF function. The double stranded oligonucleotides used to effect RNAi are preferably less than 30 base pairs in length and, more preferably, comprise about 25, 24, 23, 22, 21, 20, 19, 18 or 17 base pairs of ribonucleic acid. Optionally the dsRNA oligonucleotides of the invention may include 3' overhang ends. The specific sequence utilized in design of the oligonucleotides may be any contiguous sequence of nucleotides contained within the expressed gene message of the target. Several different types of molecules have been used effectively in the RNAi technology including small interfering RNA (siRNA), microRNA (miRNA) and short hairpin RNA (shRNA). Exemplary, non-limiting, siRNAs for PD-ECFG include commercially available PD-ECGF siRNAs from Santa Cruz Biotechnology (Catalog Nos. sc-72027, sc-72027-PR, sc-39697 and sc-39697-PR) and from Life Technologies (Catalos Nos. 117017, 119448 and 119449. Exemplary, non-limiting, shRNAs for PD-ECFG include commercially available PD-ECGF shRNAs from Santa Cruz Biotechnology (Catalog Nos. sc-72027-SH and sc-72027-V).

In a particular embodiment, the PD-ECGF inhibitor for use according to the invention is a ribozyme molecules designed to catalytically cleave a target mRNA transcripts can also be used to prevent translation of PD-ECGF mRNAs. Accordingly, in another embodiment, the PD-ECGF inhibitor for use according to the invention comprises a ribozyme specifically directed to the PD-ECGF mRNA.

In a particular embodiment, the PD-ECGF inhibitor for use according to the invention is a PD-ECGF-specific antisense nucleic acid, i.e. a nucleic acid capable of inhibiting transcription and/or translation of PD-ECGF nucleic acid.

In a particular embodiment, the PD-ECGF inhibitor for use according to the invention is a PD-ECGF-specific DNA enzyme.

The PD-ECGF inhibitor for use according to the invention is aimed at the treatment of anti-angiogenic treatment-resistant cancer in a subject.

In a particular embodiment, the anti-angiogenic treatment-resistant cancer comprises lung cancer, sarcoma, malignant melanoma, pleural mesothelioma, bladder carcinoma, prostate cancer, pancreas carcinoma, gastric carcinoma, ovarian cancer, hepatoma, breast cancer, colorectal cancer, kidney cancer, esophageal cancer, suprarenal cancer, parotid gland cancer, head and neck carcinoma, cervix cancer, endometrial cancer, liver cancer, mesothelioma, multiple myeloma, leukaemia, and lymphoma. In a particular embodiment, the anti-angiogenic treatment-resistant cancer is kidney cancer or breast cancer.

In a preferred embodiment, the cancer resistant to angiogenic treatment is a cancer which is resistant to, without limitation, anti-VEGF agents, including monoclonal antibodies such as bevacizumab (Avastin, a recombinant humanized monoclonal IgG1 antibody that binds to and inhibits the biological activity of human VEGFA in *in vitro* and *in vivo* assay systems), antibody derivatives such as ranibizumab (Lucentis), or antibody fragments such as Fab IMC 1121 or F200 Fab or orally-available small molecules that inhibit the tyrosine kinases stimulated by VEGF such as lapatinib (Tykerb), sunitinib (Sutent), sorafenib (Nexavar), axitinib, and pazopanib; anti-fibroblast growth factor (anti-FGF) agents, such as suramin and its derivatives, pentosan polysulfate, cediranib, pazopanib, or BIBF 1120); anti-EGF agents, such as cetuximab, gefitinib or erlotinib and anti-HGF agents, such as ARQ197, JNJ-38877605, PF-04217903, SGX523, NK4, or AMG102; and antiangiogenic polypeptides such as angiostatin, endostatin, anti-angiogenic anti-thrombin III or sFRP- 4.

Further cancer resistant to angiogenic treatment are cancer which are resistant to anti-angiogenic agents such as Marimastat; AG3340; COL-3, BMS-275291, Thalidomide, Endostatin, SU5416, SU6668, EMD121974, 2-methoxyoestradiol, carboxiamidotriazole, CMIOI (GBS toxin), pentosan polysulphate, angiopoietin 2 (Regeneron), herbimycin A, PNU145156E, 16K prolactin fragment, Linomide, thalidomide, pentoxifylline, genistein, TNP470, endostatin, paclitaxel, accutin, angiostatin, cidofovir, vincristine, bleomycin, AGM- 1470, platelet factor 4 or minocycline.

Further cancer resistant to angiogenic treatment are cancers which are resistant to anti-angiogenic agents such as anti-angiogenic polypeptides, denoting polypeptides capable of inhibiting angiogenesis and including, without limitation, angiostatin, endostatin, anti-angiogenic anti-thrombin III, sFRP- 4 as described in WO2007115376, an anti-VEGF antibody such as anibizumab, bevacizumab (avastin), Fab IMC 1121 and F200 Fab.

Further cancer resistant to angiogenic treatment are cancers which are resistant to anti-angiogenic agents such pegaptanib, sunitinib, sorafenib, vatalanib and aflibercept (VEGF-Trap).

Further cancer resistant to angiogenic treatment are cancers which are resistant to anti-angiogenic agents such VEGFR2 blocking antibodies, such as DC101 (also known as anti-Flk-1 mAb).

In a particular embodiment, the cancer resistant to angiogenic treatment is a cancer selected from the group comprising sunitinib, bevacizumab, and DC101.

A suitable assay to determine whether a cancer is a resistant to anti-angiogenic treatment by a particular agent is, for example, any method suitable for detecting tumor preogression after the tumor has been treated with an antiangiogenic agent, such as, for instance, tumor progression by international RECIST guidelines (a >20% increase in the sum of the longest diameter of target lesions from diagnostic imaging techniques) or a unequivocal clinical progression of disease as determined by different clinical criteria (Motzer et al., New Engl. J. Med., 2007, 356:115-124; Escudier et al. 2007, Lancet; 370: 2103-11 and Heng et al. Annals Oncology, 2011, 23: 1549-1555).

As the skilled person acknowledges, effectiveness of a cancer therapy is demonstrated by reduced tumor volume, improved symptoms, and/or decreased serological tumor markers. However, after a variable period, progression due to therapy resistance may occur. Tumor resistance, such as kidney tumor resistance, to an anti-angiogenic agent-based treatment can develop as *de novo* resistance, wherein the tumor is not responsive to the therapy, or as acquired resistance, wherein, after initial response to the therapy, the tumor develops resistance leading to disease progression.

In the context of the invention, the PD-ECGF inhibitors as described previously are used in the treatment of cancer, wherein the cancer is resistant to an anti-angiogenic agent. In a particular embodiment, the PD-ECGF inhibitors as described previously are used in the treatment of cancer, wherein the cancer is resistant to an anti-angiogenic agent by intrinsic or by acquired resistance to said agent.

Various techniques have been developed in the art for monitoring tumor response to a therapy, wherein measuring tumor size and/or shrinkage on computed thomography (CT) or other anatomic imaging modalities represents a current standard. Imaging of tumor metabolism with PET and the glucose analog 18F-FDG is contemplated as well for assessing the effects of therapy objectively and quantitatively. In cases where the tumor can be easily accessed, biopsies may be taken sequentially before and during treatment and analyzed to monitor the effects of treatment on molecular processes, including development of tumor resistance. Methods for obtaining a biopsy sample are known by the skilled person and include, without limitation, surgical resection of a tissue mass or microdissection or other known cell separation methods, the cells can additionally be obtained by aspiration cytology by means of the pricking with a thin needle connected to a syringe.

### Compositions of the invention

In a further aspect, the invention relates to a composition comprising a PD-ECGF inhibitor and an anti-angiogenesis agent.

PD-ECGF inhibitors according to the invention have been previously described. In a particular embodiment, the PD-ECGF inhibitor of the composition of the invention is selected from the group comprising an anti-PD-ECGF antibody, a PD-ECGF specific RNA interference (RNAi), a PD-ECGF specific ribozyme, a PD-ECGF specific antisense nucleic acid, a PD-ECGF specific DNA enzyme, and a PD-ECGF inhibitor according to Table 1. In a more particular embodiment, the PD-ECGF inhibitor of the composition of the invention is selected from the group comprising KIN59 and AEAC.

Anti-angiogenesis agents according to the invention have been previously described as well.

In a preferred embodiment, anti-angiogenesis agents are, without limitation, anti-VEGF agents, including VEGF-specific antibodies monoclonal antibodies such as bevacizumab (Avastin, a recombinant humanized monoclonal IgG1 antibody that binds to and inhibits the biological activity of human VEGFA in *in vitro* and *in vivo* assay systems), antibody derivatives such as ranibizumab (Lucentis), or antibody fragments such as Fab IMC 1121 or F200 Fab or orally-available small molecules that inhibit the tyrosine kinases stimulated by VEGF such as lapatinib (Tykerb), sunitinib (Sutent), sorafenib (Nexavar), axitinib, and pazopanib; anti-fibroblast growth factor (anti-FGF) agents, such as suramin and its derivatives, pentosan polysulfate, cediranib, pazopanib, or BIBF 1120); anti-EGF agents, such as cetuximab, gefitinib or erlotinib and anti-HGF agents, such as ARQ197, JNJ-38877605, PF-04217903, SGX523, NK4, or AMG102; and antiangiogenic polypeptides such as angiostatin, endostatin, anti-angiogenic anti-thrombin III or sFRP- 4.

Further anti-angiogenesis agents include inhibitors of the tyrosine kinase activity of the VEGFR, such as Marimastat; AG3340; COL-3, BMS-275291, Thalidomide, Endostatin, SU5416, SU6668, EMD121974, 2-methoxyoestradiol, carboxiamidotriazole, CMIOI (GBS toxin), pentosan polysulphate, angiopoietin 2 (Regeneron), herbimycin A, PNU145156E, 16K prolactin fragment, Linomide, thalidomide, pentoxifylline, genistein, TNP470, endostatin, paclitaxel, accutin, angiostatin, cidofovir, vincristine, bleomycin, AGM- 1470, platelet factor 4 or minocycline.

Further anti-angiogenesis agents are anti-angiogenic polypeptides, denoting polypeptides capable of inhibiting angiogenesis and including, without limitation, angiostatin, endostatin, anti-angiogenic anti-thrombin III, sFRP- 4 as described in WO2007115376, an anti-VEGF antibody such as anibizumab, bevacizumab (avastin), Fab IMC 1121 and F200 Fab.

Further anti-angiogenesis agents are pegaptanib, sunitinib, sorafenib, vatalanib and aflibercept (VEGF-Trap).

Further anti-angiogenesis agents include, without limitation, VEGFR2 blocking antibodies, such as DC101 (also known as anti-Flk-1 mAb).

In a particular embodiment, the anti-angiogenesis agents is selected from the group comprising sunitinib, bevacizumab, and DC101.

Assays to determine the antiangiogenic activity of a particular agent are described, without limitation, in WO 2003086299.

In a particular embodiment, the anti-angiogenic agent is selected from the group comprising sunitinib, bevacizumab and DC101.

In a particular embodiment, the composition of the invention is a pharmaceutical composition, wherein said pharmaceutical composition comprises a pharmaceutically acceptable vehicle, including additives, such as preservatives, excipients, loads, wetting agents, binding agents, disintegrating agents and buffers. These additives can be, for instance, magnesium and calcium carbonates, carboxymethyl cellulose, starches, sugars, gums, magnesium or calcium stearate, colouring matters, or flavouring agents. There is a high variety of pharmaceutically acceptable additives for pharmaceutical dosage forms and the selection of appropriate additives is a routine matter for the skilled in the art of pharmaceutical formulation. Excipients or vehicles preferred for use in this invention include sugars, starches, celluloses, gums, and proteins. In a particular embodiment, the pharmaceutical composition of the invention will be formulated in a solid (e.g., tablets, capsules, lozenges, granules, suppositories, sterile crystalline or amorphous solids that can be reconstituted to provide liquid forms etc.), liquid (for instance, solutions, suspensions, emulsions, elixirs, lotions, ointments, etc.) or semisolid (gels, ointments, creams, and similar) pharmaceutical dosage form.

The term "pharmaceutical composition", as used herein, refers to a composition comprising a therapeutical effective amount of the agent according to the present invention and at least one pharmaceutically acceptable excipient. Pharmaceutical compositions according to the invention can be prepared, for instance, as injectables such as liquid solutions, suspensions, and emulsions.

The terms "pharmaceutically acceptable excipient", or "pharmaceutically acceptable carrier," "pharmaceutically acceptable diluent,", or "pharmaceutically acceptable vehicle," used interchangeably herein, refer to a non-toxic solid, semisolid or liquid filler, diluent, encapsulating material or formulation auxiliary of any conventional type. A pharmaceutically acceptable carrier is essentially non-toxic to recipients at the dosages and concentrations employed, and is compatible with other ingredients of the formulation. Suitable carriers include, but are not limited to water, dextrose, glycerol, saline, ethanol, and combinations thereof. The carrier can contain additional agents such as wetting or emulsifying agents, pH buffering agents, or adjuvants which enhance the effectiveness of the formulation. Adjuvants could be selected from the group consisting of sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

The term "therapeutical effective amount", as used herein in relation to the agent of the invention, or in relation to the agent, excipient and/or carrier comprised by the pharmaceutical composition of the invention, relates to the sufficient amount of said agent, excipient and/or carrier to provide the desired effect, i.e. to achieve an appreciable prevention, cure, delay, reduction of severity or amelioration of one or more symptoms derived from a disease, and will generally be determined by, among other causes, the characteristics of the agent itself and the therapeutic effect to be achieved. It will also depend on the subject to be treated, the severity of the disease suffered by said subject, the chosen dosage form, etc. For this reason, the doses mentioned in this invention must be considered only as guides for the person skilled in the art, who must adjust the doses depending on the aforementioned variables. In an embodiment, the effective amount produces the amelioration of one or more symptoms of the disease that is being treated.

Those skilled in the art are familiar with the principles and procedures discussed in widely known and available sources as Remington's Pharmaceutical Science (17th Ed., Mack Publishing Co., Easton, Pa., 1985) and Goodman and Gilman's The Pharmaceutical Basis of Therapeutics (8th Ed., Pergamon Press, Elmsford, N.Y., 1990) both of which are incorporated herein by reference.

In a preferred embodiment of the present invention, the compounds of the composition of the invention are formulated in accordance with standard procedure as a pharmaceutical composition adapted for delivered administration to human beings and other mammals. Typically, compositions for intravenous or intraventricular administration are solutions in sterile isotonic aqueous buffer.

Where necessary, the composition of the invention also includes a solubilizing agent and a local anesthetic to ameliorate any pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water or saline. Where the composition is administered by injection, an ampule of sterile water for injection or saline can be provided so that the ingredients may be mixed prior to administration.

In cases other than intravenous administration, the composition can contain minor amounts of wetting or emulsifying agents, or pH buffering agents. The composition can be a liquid solution, suspension, emulsion, gel, polymer, or sustained release formulation. The composition can be formulated with traditional binders and carriers, as would be known in the art. Formulations can include standard carriers such as pharmaceutical grades of mannitol, lactose, starch, magnesium stearate, sodium saccharide, cellulose, magnesium carbonate, etc., inert carriers having well established functionality in the manufacture of pharmaceuticals. Various delivery systems are known and can be used to administer a therapeutic of the present invention including encapsulation in liposomes, microparticles, microcapsules and the like.

In yet another preferred embodiment, therapeutics containing the compounds of the invention can be formulated as neutral or salt forms. Pharmaceutically acceptable salts include those formed with free amino groups such as those derived from hydrochloric, phosphoric, acetic, oxalic, tartaric acids and the like, and those formed with free carboxyl groups such as those derived from sodium, potassium, ammonium, calcium, ferric hydroxides, isopropylamine, thriethylamine, 2-ethylamino ethanol, histidine, procaine or similar.

The pharmaceutical compositions containing the compounds according to the invention can occur at any pharmaceutical form of administration considered appropriate for the selected administration route, for example, by systemic, oral, parenteral, intradermal, subcutaneous, intramuscular, intravenous or topical administration, for which it will include the pharmaceutically acceptable excipients necessary for formulation of the desired method of administration.

The effective quantity of the compounds of the invention can vary within a wide range and, in general, will vary depending on the particular circumstances of application, duration of the exposure and other considerations.

Solid dosage forms for oral administration may include conventional capsules, sustained release capsules, conventional tablets, sustained-release tablets, chewable tablets, sublingual tablets, effervescent tablets, pills, suspensions, powders, granules and gels. At these solid dosage forms, the active compounds can be mixed with at least one inert excipient such as sucrose, lactose or starch. Such dosage forms can also comprise, as in normal practice, additional substances other than inert diluents, e.g. lubricating agents such as magnesium stearate. In the case of capsules, tablets, effervescent tablets and pills, the dosage forms may also comprise buffering agents. Tablets and pills can be prepared with enteric coatings.

Liquid dosage forms for oral administration may include emulsions, solutions, suspensions, syrups and elixirs pharmaceutically acceptable containing inert diluents commonly used in the technique, such as water. Those compositions may also comprise adjuvants such as wetting agents, emulsifying and suspending agents, and sweetening agents, flavoring and perfuming agents.

Injectable preparations, for example, aqueous or oleaginous suspensions, sterile injectable may be formulated according with the technique known using suitable dispersing agents, wetting agents and/or suspending agents. Among the acceptable vehicles and solvents that can be used are water, Ringer's solution and isotonic sodium chloride solution. Sterile oils are also conventionally used as solvents or suspending media.

For topical administration, compounds of the invention can be formulated as creams, gels, lotions, liquids, pomades, spray solutions, dispersions, solid bars, emulsions, microemulsions and similars which may be formulated according to conventional methods that use suitable excipients, such as, for example, emulsifiers, surfactants, thickening agents, coloring agents and combinations of two or more thereof.

Additionally, the compounds of the invention may be administered in the form of transdermal patches or iontophoresis devices. In one embodiment, the compounds of the invention are administered as a transdermal patch, for example, in the form of sustained-release transdermal patch. Suitable transdermal patches are described in more detail in, for example, US5262165, US5948433, US6010715 and US6071531.

The compositions comprising the compounds of the invention can additionally include conventional excipients, i.e. pharmaceutically acceptable carriers suitable for parenteral application which do not react damaging with the active compounds. Suitable pharmaceutically acceptable vehicles include, for example, water, salt solutions, alcohol, vegetable oils, polyethylene glycols, gelatin, lactose, amylose, magnesium stearate, talc, surfactants, silicic acid, viscous paraffin, perfume oil, monoglycerides and diglycerides of fatty acids, fatty acid esters petroetrals, hydroxymethyl cellulose, polyvinylpyrrolidone and similars.

Several drug delivery systems are known and can be used to administer the compounds or compositions of the invention, including, for example, encapsulation in liposomes, microbubbles, emulsions, microparticles, microcapsules and similars. The required dosage can be administered as a single unit or in a sustained release form.

Sustainable-release forms and appropriate materials and methods for their preparation are described in, for example, "Modified-Release Drug Delivery Technology", Rathbone, M. J. Hadgraft, J. and Roberts, M. S. (eds.), Marcel Dekker, Inc., New York (2002), "Handbook of Pharmaceutical Controlled Release Technology", Wise, D. L. (ed.), Marcel Dekker, Inc. New York, (2000). In one embodiment of the invention, the orally administrable form of a compound according to the invention is in a sustained release form further comprises at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of the them.

Enteric coatings may be applied using conventional processes known to experts in the art, as described in, for example, Johnson, J. L., "Pharmaceutical tablet coating", Coatings Technology Handbook (Second Edition), Satas, D. and Tracton, A. A. (eds), Marcel Dekker, Inc. New York, (2001), Carstensen, T., "Coating Tablets in Advanced Pharmaceutical Solids", Swarbrick, J. (ed.), Marcel Dekker, Inc. New York (2001), 455-468.

Even though individual needs vary, determination of optimal ranges for effective amounts of the compound of the invention belongs to the common experience of those experts in the art. In general, the dosage needed to provide an effective amount of such compound, which can be adjusted by one expert in the art will vary depending on age, health, fitness, sex, diet, weight, degree of alteration of the receptor, frequency of treatment and the nature and extent of impairment or illness, medical condition of the patient, route of administration, pharmacological considerations such as activity, efficacy, pharmacokinetic and toxicology profile of the particular compound used, if using a system drug delivery, and if the compound is administered as part of a combination of drugs.

The amount of the compound according to the invention that is effective in the treatment of a particular disorder or condition will depend on the nature of the disorder or condition, in particular breast cancer, and can be determined by conventional clinical techniques, including reference to Goodman and Gilman, supra; The Physician's Desk Reference, Medical Economics Company, Inc., Oradell, NJ, 1995, and Drug Facts and Comparisons, Inc., St. Louis, MO, 1993. The precise dose to use in the formulation will also depend on the route of administration, and severity of the disease or disorder, and should be decided in the physician's opinion and the patient's circumstances.

### Methods for the treatment of anti-angiogenic treatment-resistant cancer

In a further aspect, the invention relates to a composition as previously described or to a pharmaceutical composition as previously described comprising a PD-ECGF inhibitor and an anti-angiogenesis agent for use in the treatment of cancer, wherein said cancer is resistant to anti-angiogenic treatment. Alternatively, the invention relates to a composition or pharmaceutical composition as previously described in the manufacture of a medicament for the treatment of cancer, wherein said cancer is resistant to an anti-angiogenic treatment. Alternatively, the invention relates to a method for the treatment of cancer in a subject in need thereof that comprises the administration of a therapeutically effective amount of a composition or pharmaceutical composition according to the invention to a subject suffering from cancer, wherein said cancer is resistant to an anti-angiogenic treatment.

Appropriate amounts of compounds according to the present invention can be formulated with pharmaceutically acceptable excipients and/or carriers to obtain a pharmaceutical composition for use in the treatment of cancer, wherein said cancer is resistant to an anti-angiogenic treatment.

As a person skilled in the art understands, the composition of the invention for use in the treatment of cancer, wherein said cancer is resistant to an anti-angiogenic treatment, will be formulated according to the administration route to be used.

The administration of the composition of the invention can be performed by different routes, for instance, intravenous, intraperitoneal, subcutaneous, intramuscular, topical, intradermal, intranasal or intrabronchial, and it can be administered locally or systemically or directly to the target site. A review of the different administration routes of active substances, of the excipients to be used and the manufacturing procedures can be found in Tratado de Farmacia Galénica, C. Faulí i Trillo, Luzán 5, S.A. de Ediciones, 1993 and in Remington's Pharmaceutical Sciences (A.R. Gennaro, Ed.), 20th edition, Williams & Wilkins PA, USA (2000).

The dosage regimen will be established by the physician and the clinical factors. As it is well known in medicine, the dosages depend on many factors, including the physical characteristics of the patient (age, size, sex), the administration route used, the severity of the disease, the particular compound used and the pharmacokinetic properties of the subject.

In a particular embodiment, the cancer is kidney cancer or breast cancer.

Methods to evaluate cancer resistance to a treatment, such as an anti-angiogenic treatment, as well as methods to monitor tumor response to a treatment, have been described in the context of the compounds of the invention for their use in the treatment of anti-angiogenic treatment-resistant cancer.

### Methods for determining tumor resistance or sensitivity to anti-angiogenic treatment

The inventors have found that the expression levels of PD-ECGF are particularly increased in those patients showing acquired resistance to an anti-angiogenic treatment when compared to the PD-ECGF expression levels in patients showing intrinsic resistance to an anti-angiogenic treatment or to patients responding to an anti-angiogenic treatment (see Example 4, Figure 13c). Thus, in a further aspect, the invention is related to an *in vitro* method for determining whether a tumor in a patient is resistant or sensitive to an anti-angiogenesis agent that comprises
(i) determining the expression level of PD-ECGF in a sample from said patient, and
(ii) comparing the expression level in (i) to a reference value,
wherein an increased expression level of PD-ECGF is indicative that the tumor is resistant to an anti-angiogenesis agent, and a decreased expression level of PD-ECGF is indicative that the tumor is sensitive to an anti-angiogenesis agent.

Thus, in a first step of the method of the invention, the expression levels of PD-ECGF1 are determined in a sample from the patient. The sample wherein the expression level of PD-ECGF1 is determined can be any sample containing cells from the tumor. In a particular embodiment, the sample containing cells from the tumor is tumor tissue or a portion thereof. In a more particular embodiment, said tumor tissue sample is a kidney tumor tissue sample from a patient suffering from kidney cancer, or a breast tumor tissue sample from a patient suffering from breast cancer. Said sample can be obtained by conventional methods, e.g., biopsy, surgical excision or aspiration, by using methods well known to those of ordinary skill in the related medical arts. Methods for obtaining the sample from the biopsy include gross apportioning of a mass, or microdissection or other art-known cell-separation methods including nephrectomy and partial tumorectomy. Tumor cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze.

In another embodiment, the sample wherein the expression level of PD-ECGF1 is determined is a biofluid from the patient. In a preferred embodiment, the biofluid is selected from blood, particularly peripheral blood, or serum. The blood sample is typically extracted by means of puncturing an artery or vein, normally a vein from the inner part of the elbow or from the back of the hand, the blood sample being collected in an air-tight vial or syringe. A capillary puncture normally on the heel or on the distal phalanxes of fingers can be performed for analysis by means of a micromethod. Serum can be obtained from the complete blood sample and in the absence of anticoagulant by leaving the sample to settle for 10 minutes so that it coagulates and subsequently centrifuging it at 1,500 rpm for 10 minutes for the purpose of separating the cells (precipitate) from the serum (supernatant).

As previously described, gene expression levels can be quantified by measuring the messenger RNA levels of the gene or of the protein encoded by said gene or of the protein encoded by said gene, i.e. PD-ECGF protein or of variants thereof. PD-ECGF protein variants include all the physiologically relevant post-translational chemical modifications forms of the protein, for example, glycosylation, phosphorylation, acetylation, etc., provided that the functionality of the protein is maintained. Said term encompasses the PD-ECGF protein of any mammal species, including but not being limited to domestic and farm animals (cows, horses, pigs, sheep, goats, dogs, cats or rodents), primates and humans. Preferably, the PD-ECGF protein is a human protein.

In order to measure the mRNA levels of a gene, the biological sample may be treated to physically, mechanically or chemically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor. In a particular embodiment samples can be obtained from fresh tumor tissue or from OCT embedded frozen tissue.

In order to normalize the values of mRNA expression among the different samples, it is possible to compare the expression levels of the mRNA of interest in the test samples with the expression of a control RNA. A "control RNA" as used herein, relates to RNA whose expression levels do not change or change only in limited amounts in tumor cells with respect to non-tumorigenic cells. Preferably, the control RNA is mRNA derived from housekeeping genes and which code for proteins which are constitutively expressed and carry out essential cellular functions. Preferred housekeeping genes for use in the present invention include β-2-microglobulin, ubiquitin, 18-S ribosomal protein, cyclophilin, IPO8, HPRT, GAPDH, PSMB4, tubulin and β-actin. In a preferred embodiment, the control RNA is GAPDH, IPO8, HPRT, β-actin, 18-S ribosomal protein or PSMB4 mRNA.

In one embodiment relative gene expression quantification is calculated according to the comparative threshold cycle (Ct) method using GAPDH, IPO8, HPRT, β-actin or PSMB4 as an endogenous control and commercial RNA controls as calibrators. Final results are determined according to the formula 2-(ΔCt sample-ΔCt calibrator), where ΔCT values of the calibrator and sample are determined by subtracting the Ct value of the target gene from the value of the control gene.

Suitable methods to determine gene expression levels at the mRNA and the protein level have been described in the context of the PD-ECGF inhibitors for use in the treatment of anti-angiogenic treatment-resistant cancer of the invention. Standard assays for determining mRNA expression levels such as qPCR, RT-PCR, RNA protection analysis, Northern blot, RNA dot blot, *in situ* hybridization, microarray technology, tag based methods such as serial analysis of gene expression (SAGE) including variants such as LongSAGE and SuperSAGE, microarrays, fluorescence in situ hybridization (FISH), including variants such as Flow-FISH, qFiSH and double fusion FISH (D-FISH), and the like.

The expression levels of PD-ECGF protein can be quantified by means of conventional methods, for example, using antibodies with a capacity to specifically bind to the proteins encoded by said genes (or to fragments thereof containing antigenic determinants) and subsequent quantification of the resulting antibody-antigen complexes. In a particular embodiment, PD-ECGF protein levels can be quantified by using standard assays for determining protein expression levels such as Western-blot or Western transfer, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of protein biochips or microarrays which include specific antibodies or assays based on colloidal precipitation in formats such as dipsticks.

The antibodies to be employed in these assays can be, for example, polyclonal sera, hybridoma supernatants or monoclonal antibodies, antibody fragments, Fv, Fab, Fab' and F(ab')2, ScFv, diabodies, triabodies, tetrabodies and humanized antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc. There are a wide variety of well-known assays that can be used in the present invention, which use non-labeled antibodies (primary antibody) and labeled antibodies (secondary antibodies); among these techniques are included Western blot or Western transfer, ELISA, RIA, competitive EIA, DAS-ELISA, immunocytochemical and immunohistochemical techniques, techniques based on the use of biochips or protein microarrays including specific antibodies or assays based on colloidal precipitation in formats such as dipsticks. Other ways of detecting and quantifying the levels of the protein of interest include techniques of affinity chromatography, binding-ligand assays, etc.

On the other hand, the determination of the levels of the PD-ECGF protein can be carried out by constructing a tissue microarray (TMA) containing the subject samples assembled, and determining the expression levels of the corresponding protein by immunohistochemistry techniques. Immunostaining intensity can be evaluated by two or more different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumor cells and the specific cut-off for each marker. As a general criterion, the cut-offs are selected in order to facilitate reproducibility, and when possible, to translate biological events. Alternatively, the immunostaining intensity can be evaluated by using imaging techniques and automated methods such as those disclosed in Rojo, M.G. et al. (Folia Histochem. Cytobiol. 2009; 47: 349-54) or Mulrane, L. et al. (Expert Rev. Mol. Diagn. 2008; 8: 707-25).

Alternatively, in another particular embodiment, the levels of the PD-ECGF protein are determined by Western blot. Western blot is based on the detection of proteins previously resolved by gel electrophoreses under denaturing conditions and immobilized on a membrane, generally nitrocellulose, by the incubation with an antibody specific and a developing system (e.g. chemoluminiscent).

The term "activity level" of a protein, more particularly of an enzyme, as used herein refers to a measure of the enzyme activity, particularly measured as moles of substrate converted per unit of time.

Assays to determine the activity level of an enzyme are known by the skilled person and include, without limitation, initial rate assays, progress curve assays, transient kinetics assays and relaxation assays. Continuous assays of enzymatic activity include, without limitation, spectrophotometric, fluorometric, calorimetric, chemiluminiscent, light scattering and microscale thermopheresis assays. Discontinuous assays of enzymatic activity include, without limitation, radiometric and chromatographic assays. As the skilled person understands, factors that may influence enzymatic activity comprise salt concentration, temperature, pH, and substrate concentration.

In a second step of the method of the invention, the expression levels of PD-ECGF in the sample are compared to a reference value.

In the context of the method of the invention for determining tumor resistance to an anti-angiogenic agent, the reference value is the PD-ECGF expression level determined in a sample from a healthy subject, a subject not suffering from cancer, or a tumor sample from a subject suffering from cancer wherein said tumor is sensitive to an anti-angiogenic therapy, preferably the reference value is the PD-ECGF expression level determined in a tumor sample from a subject suffering from cancer wherein said tumor is sensitive to an anti-angiogenic therapy.

In the context of the method of the invention for determining tumor sensitivity to an anti-angiogenesis agent, the reference value is the PD-ECGF expression level determined in tumor sample from a subject suffering from cancer wherein said tumor is resistant to an anti-angiogenic factor.

Once this reference value is established, the level of PD-ECGF expressed in the sample can be compared with said reference value, and thus be assigned a level of "increased" or "decreased" expression. For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level.

Thus, an increased expression level of PD-ECGF in the sample when compared to a reference value is indicative that the tumor cell is resistant to an anti-angiogenesis agent. Alternatively, a decreased expression level of PD-ECGF in the sample when compared to the reference value is indicative that the tumor cell is sensitive to an anti-angiogenesis agent.

Anti-angiogenesis agents according to the invention have been described previously in the context of the PD-ECGF inhibitors of the invention for use in the treatment of anti-angiogenic treatment-resistant cancer. In a particular embodiment, the anti-angiogenesis agent is selected from the group comprising sunitinib, bevacizumab and DC101.

The invention is described below by the following examples, which must be considered as merely illustrative and in no case limiting of the scope of the present invention.

### EXAMPLES

### Example 1. Identification of PD-ECGF as a potential marker of resistance to anti-angiogenic agents

The inhibition of angiogenesis in the VEGF/VEGFR pathway results in resistance in a mouse model of renal cancer derived from cell lines and from biopsies of primary tumors from patients.

By means of a human low density array, the inventors studied the molecular pathway that was involved in the process of resistance and that may function as a possible escape route to the anti-angiogenic therapy.

The results showed that a number of genes were overexpressed or downregulated by the anti-angiogenic therapy. In particular, PD-ECGF was overexpressed, with a fold-change of 300 in control tumors *vs*. treated tumors.

In order to confirm the array data, an *in vivo* analysis based on immunohistochemistry was performed to determine whether PD-ECGF was increased after treatment with anti-angiogenic agents.

A significant increase in the expression of PD-ECGF was observed in anti-angiogenesis treated tumors when compared to controls, wherein the tumors were derived from human renal cell carcinoma 7860- cell line and the anti-angiogenic treatment was followed for 2 weeks (Figure 1A). Similar results were observed in tumors derived from biopsies of primary tumors REN 13 (Figure 1B), REN 28 (Figure 1C), REN 38 (Figure 1D) and REN 50 (Figure IE) and under anti-angiogenic treatment for 4, 3, 4.5 and 1 week(s), respectively.

### Example 2. Validation of PD-ECGF overexpression in anti-angiogenic treatment-resistant tumors

The inventors analyzed then whether the increase in PD-ECGF expression was maintained during the continuous administration of anti-angiogenic drugs (resistance phase).

PD-ECGF expression was measured in control tumors, anti-angiogenic resistant-tumors derived from 7860- cell line (Figure 2A) and anti-angiogenic resistant-tumors derived from REN 28 (primary tumor) (Figure 2B).

A significant increase in the expression of PD-ECGF was observed in 7860-derived and REN 28-derived treated tumors compared to control tumors, wherein said tumors have become resistant to treatment (survival test).

It must be taken into account that, when there is a tumor regrowth due to resistance to the treatment, the increase in PD-ECGF expression is not uniform along the tumor, and that different areas with positive expression were observed both in anti-angiogenic-treated tumors and control tumors.

Additionally, the inventors confirmed the previous results at the protein level by performing an ELISA (Figure 3). PD-ECGF protein levels were increased in tumor cells under DC101 treatment compared to control tumors.

### Example 3. Inhibition of PD-ECGF as a therapeutic target in anti-angiogenic resistant renal tumors

After *in vivo* experimental results showing PD-ECGF deregulation in both RNA and protein levels, inhibitors of PD-ECGF enzymatic activity were assayed in renal tumors.

Agents selectively inhibiting the enzymatic activity of PD-ECGF were selected, based on 5'-O-trityl-inosine (KIN59) and on 6-(2-aminoethyl)amino-5-chlorouracil (AEAC).

### 3.1. PD-ECGF inhibition showed effectiveness in renal cell carcinoma tumors

An effectiveness experiment was performed according to the following scheme: tumors of about 2 mm³ size were implanted in mice, and the tumors were allowed to develop for 20 days (to a size of about 750 mm³). The time from tumor development to death was measured as the anti-angiogenic response.

7 mice were implanted per treatment group. REN 50 cells were implanted. Treatment started when mice developed tumors with a volume tumor of about 750 mm³, and lasted for three weeks. Treatment groups are shown in Table 2.

**Table 2. Treatment groups**

| Group | Administration | Dose | Administration frequency |
|---|---|---|---|
| Control (vehicle 10% DMSO + 10% TWEEN) | Intraperitoneal | 1ml | daily |
| DC101 | Intraperitoneal | 4mg/kg | twice a week |
| BEVACIZUMAB | Intraperitoneal | 5mg/kg | twice a week |
| KIN 59 | Intraperitoneal | 50mg/kg | daily |
| AEAC | Oral | 50mg/kg | daily |
| DC101 + KIN 59 | Intraperitoneal | DC101: 4mg/kg | DC101: twice a week |
| | | KIN 59: 50mg/kg | KIN 59: daily |
| DC101+AEAC | Intraperitoneal/ Oral | DC101: 4mg/kg | DC101: twice a week |
| | | AEAC: 50mg/kg | AEAC 59: daily |

Tumor reduction was observed with all treatments (Figure 4).

The inventors then analyzed whether these inhibitors produced any effect at the level of tumor vasculature by conducting in immunohistochemistry, using an anti-CD31 antibody as a marker of endothelial cells. The results showed that an effect at the vascular density level occurred in all treatment groups, with a significant decrease in vascular density in treated groups compared to the control group (Figures 5 and 6).

Hematoxylin-eosin stained paraffin sections of tumors obtained from mice from the different treatment groups were analyzed in order to evaluate the degree of necrosis. A statistically significant increase in the percentage of necrosis was observed in all treatment groups when compared to the control group (Figure 7).

In summary, the inventors found that there is a comparable effectiveness between PD-ECGF inhibitors and anti-angiogenic therapies (bevacizumab and DC101) used in this study. The inhibition of PD-ECGF pathway by the use of chemical inhibitors such as AEAC and KIN 59 after continuous administration resulted in decreased tumor weight and volume, decreased tumor vasculature and increased tumor necrosis. Thus, the inventors concluded that the PD-ECGF pathway is directly related to the angiogenic process.

### 3.2. Inhibition of PD-ECGF showed effectiveness in tumors resistant to anti-angiogenic therapy

The inventors assessed next the effect of therapy with PD-ECGF inhibitors in a long term survival experiment, wherein the tumors are able to regrow (resistance) and wherein PD-ECGF overexpression has been observed.

A survival experiment was followed, wherein the drugs previously tested on the primary tumor from REN 28 were continuously administered. This tumor line was selected because the inventors this tumor is able to generate an anti-angiogenic resistance phenotype with after treatment with bevacizumab and DC101.

Again, tumors of about 2 mm³ size were implanted in mice, and the tumors were allowed to develop for 30 days (with a size of about 1,000 mm³). The time from tumor development to death was measured as the anti-angiogenic resistance.

7/8 mice were implanted per treatment group, and the treatment started when he mice had an estimated tumor volume of 1,000 mm³. Mice were sacrificed when they showed symptoms of cachexia, significant weight loss or overall poor condition. Tumors were measured and weighted; samples were collected for further processing. Treatment groups were as shown in Table 3. Treatments were stopped or changed on day 15.

**Table 3. Treatment groups.**

| Group | Administration | Dose | Administration frequency |
|---|---|---|---|
| Control (vehicle 10% DMSO + 10% TWEEN) | Oral | 250µl | daily |
| DC101 | Intraperitoneal | 4mg/kg | twice a week |
| BEVACIZUMAB | Intraperitoneal | 5mg/kg | twice a week |
| AEAC | Oral | 50mg/kg | daily |
| DC101→R→OFF | Intraperitoneal | 4mg/kg | twice a week |
| DC101→R→AEAC | Intraperitoneal | 4mg/kg | twice a week |
| | Oral | 50mg/kg | daily |
| DC101→R→DC101 + AEAC | Intraperitoneal | 4mg/kg | twice a week |
| | Oral | 50mg/kg | daily |

### Survival of mice under continuous treatment with DC101, bevacizumab and AEAC

Mice under treatment with DC101, bevacizumab and AEAC showed an increased average and total survival compared to control mice (Figure 8 A-C). The three therapies showed anti-tumor effectiveness, but the bevacizumab treatment group showed a more prolonged effect, with increased time to progression and median overall in comparison to the other treatments.

Similar results were obtained in the determination of the tumor volume at the end point of the experiment (Figure 8D). A decreased number of vessels (labeled with anti-CD31) was observed after 21 days of continuous treatment with antiangiogenics (Figure 8E). A similar pattern of necrosis was observed in mice under the different treatments (Figure 8F).

These results confirmed that an inhibition of angiogenesis via the VEGF/VEGFR pathway and via the PD-ECGF pathway have an antitumor and an antiangiogenic activity, that therapy based on bevacizumab is the most effective since it increases the time in which tumors began to regrow and increases survival as well. However, it should be noted that tumors also become resistant under chemical inhibition of PD-ECGF.

### Treatment strategies in anti-angiogenic resistant tumors

Several treatment strategies were followed in anti-VEGFR2 resistant tumors:
1. stop the anti-VEGFR2 (DC101) treatment just when the tumors begin to becomes resistant, and verify if said tumors are able to regrow with or without vasculature (represented as "DC101→R→OFF" treatment group),
2. change the VEGFR2 inhibition therapy by the inhibition of another pathway (PD-ECGF) (represented as "DC101→R→AEAC" treatment group),
3. combine the VEGFR2-inhibition therapy (DC101) and the PD-ECGF-inhibition therapy (DC101) (represented as "DC101→R→DC101 1 + AEAC" treatment group).

It was shown that all treatments resulted in an increased average and overall mice survival (Figure 9A-B). Tumor volume data showed treatment effectiveness followed by resistance and then regrowth of tumors generated from the primary tumor REN 28 (Figure 9C). As shown below: 1.removal of anti-angiogenic therapy resulted in faster tumor regrowth and greater tumor volume, similar to those of control animals. 2. Addition of AEAC therapy to DC101 therapy became toxic for animals, since they died having a small tumoral volume, probably due to toxicity of drug combination. 3. Changing DC101 therapy to AEAC therapy delayed the time of tumor progression and increased survival.

Animal survival in the treatment groups "DC101" and "DC101→R→OFF" and control mice was then compared, showing that survival was similar in these two treatment groups (Figure 10A). However, when the tumor weight at the end point of the experiment was measured, it was shown that treatment stop caused tumors to grow to a similar level to those in the control group (Figure 10B). A decrease in vessels number was observed in animals under DC101 continuous treatment, while tumor revascularization was observed in the group where the treatment was stopped (Figure 10C). Results showed that the removal of the anti-angiogenic treatment results in faster tumor regrowth and revascularization.

The inventors assayed next the effect on tumor resistance and animal survival of inhibiting the PD-ECGF pathway when anti-angiogenic resistance started to develop, in tumors under DC101 treatment, as well as the effect of inhibiting the PD-ECGF pathway instead the VEGR2 pathway.

When tumors were treated with a combination of DC101 and AEAC after development of resistance, a decreased survival was observed in the group of animals under combined therapy (Figure 11A). This effect could be due to toxicity of the combination since animals showed signs of weight loss and cachexia with small tumor size when sacrificed (Figure 11B). A significant decrease in the number of vessels after treatment was observed in both groups (Figure 11C).

Thus, although the addition of AEAC to DC 101 therapy is toxic to animals, it must be considered the sustained effect on tumor volume reduction and decreased vascular density, which indicates that inhibition of both PD-ECGF and VEGFR-2 pathways could delay the acquisition of resistance to therapy if the problems of toxicity are overcome.

The inventors assayed next the effect on tumor resistance if the pathway to be inhibited is changed when resistance is developed. If at the beginning of tumor resistance development, the therapy is changed from inhibition of VEGF pathway to inhibition of PD-ECGF pathway, animal survival slightly increases (Figure 12A), tumor weight shows high variability (Figure 12B) and tumor vasculature is reduced (Figure 12C). Thus, changing inhibition of the VEGF pathway for the PD-ECGF pathway delayed the time of tumor progression and increased survival, which indicates that inhibiting a pathway other than the VEGF pathway at the onset of resistance, could delay the development of said resistance and tumor progression.

### Example 4. PD-ECGF as predictor of the response to anti-angiogenic therapy and as prognostic factor

PD-ECGF expression was analyzed in primary tumors from RCC and breast cancer patients by immunodetection. A mouse monoclonal anti-thymidime phosphorylase [P-GF, 44C] antibody was used (Abcam, ab 3151).

Results showed that:
- PD-ECGF was expressed in all primary tumors analyzed,
- PD-ECGF has different locations (membrane, cytoplasm and nucleus), which allows evaluating the different degrees of expression according to location, and
- PD-ECGF shows different expression levels depending on the intrinsic characteristics of each tumor.

When PD-ECGF basal expression levels were analyzed, it was observed that the tumors showed different basal expression levels (Figure 13A), probably depending on the intrinsic characteristics of each tumor.

To assess whether PD-ECGF could have a value as predictor of resistance to an anti-angiogenic treatment or as a pre-treatment prognostic factor, pre- and post-anti-angiogenic treatment paired renal tumor samples from patients of the Hospital de Bellvitge and Hospital Vall d'Hebron were analyzed.

An immunohistochemistry of PD-ECGF in pre- and post-treatment samples from responders and refractory patients was performed. The results are shown in Table 4.

**Table 4. PD-ECGF expression levels in RCC Cancer patients before (Mx PRE) and after (Mx POST) the anti-angiogenic treatment**

| **Patient** | **Progression** | **Mx PRE** | | **Mx POST** | | **fold change** |
|---|---|---|---|---|---|---|
| | | **PD-ECGF** | **SD** | **PD-ECGF** | **SD** | |
| Patient 1 | No/ in response | 315 | 5 | 215 | 5 | **0,68** |
| Patient 2 | No/ in response | 276,7 | 5,8 | 300 | 0 | **1,08** |
| Patient 3 | No/ in response | 218,3 | 2,9 | 344 | 5,8 | **1,58** |
| Patient 4 | No/ in response | 350 | 18 | 268,3 | 7,6 | **0,77** |
| Patient 5 | No/ in response | 376,7 | 5,8 | 400 | 0 | **1,06** |
| Patient 6 | Yes/in resistance | 273,3 | 23,6 | 358,3 | 2,9 | **1,31** |
| Patient 7 | Yes/in resistance | 353,3 | 7,6 | 368,3 | 7,6 | **1,04** |
| Patient 8 | Yes/in resistance | 201,7 | 14,4 | 185 | 21,7 | **0,92** |
| Patient 9 | Yes/in resistance | 178,3 | 2,9 | 318,3 | 10,4 | **1,79** |
| Patient 10 | Yes/in resistance | 111,7 | 2,9 | 150 | 10 | **1,34** |
| Patient 11 | Yes/in resistance | 265 | 21 | 380 | 5 | **1,43** |
| Patient 12 | Yes/in resistance | 220 | 5 | 360 | 0 | **1,64** |

A tendency to increased expression levels of PD-ECGF after application of the anti-angiogenic therapy was observed: from the 12 pre- and post-treatment samples tested, 9 showed increased PD-ECGF levels, and only 3 of them showed a minor decrease.

Due to the heterogeneity in terms of duration and type of treatment of the patients, they were grouped according to their progression into 2 groups: those who responded to the anti-angiogenic therapy and those who had resistance to anti-angiogenic therapy, based on whether they were or were not on progression at the moment when the second sample was obtained. The difference between the total percentage of increase and/or decrease of PD-ECGF in the patients according to their progression was quantified.

All patients groups exhibited an increase in PD-ECGF expression (Figure 13C). However, the greater percentage increase was observed in the group of patients who have resistance to anti-angiogenic therapy.

Moreover in tissue samples from breast cancer patients the expression levels of PD-ECGF are particularly increased in those patients after anti-angiogenic treatment when compared to the PD-ECGF basal expression levels. (see Figure 14).

## Claims

1. A PD-ECGF inhibitor for use in the treatment of cancer in a subject in need thereof, wherein said cancer is resistant to an anti-angiogenic treatment.

2. The inhibitor for use according to claim 1, wherein the PD-ECGF inhibitor is selected from the group comprising an anti-PD-ECGF antibody, a PD-ECGF specific RNA interference (RNAi), a PD-ECGF specific ribozyme, a PD-ECGF specific antisense nucleic acid, a PD-ECGF specific DNA enzyme, and a PD-ECGF inhibitor according to Table 1.

3. The inhibitor for use according to any of claims 1 or 2, wherein the PD-ECGF inhibitor is selected from the group comprising KIN59 or a derivative thereof and AEAC.

4. The inhibitor for use according to any of the preceding claims, wherein the resistance to the anti-angiogenic treatment is a resistance against a VEGF-targeted therapy.

5. The inhibitor for use according to claim 4 wherein the VEGF-target therapy comprises a therapy with an anti-VEGF antibody, a therapy with an anti-VEGFR antibody and a therapy with a tyrosine kinase inhibitor.

6. The inhibitor for use according to any of the preceding claims, wherein the cancer is kidney cancer or breast cancer.

7. A composition comprising a PD-ECGF inhibitor and an anti-angiogenesis agent.

8. The composition according to the preceding claim, wherein the PD-ECGF inhibitor is selected from the group comprising an anti-PD-ECGF antibody, a PD-ECGF specific RNA interference (RNAi), a PD-ECGF specific ribozyme, a PD-ECGF specific antisense nucleic acid, a PD-ECGF specific DNA enzyme, and a PD-ECGF inhibitor according to Table 1.

9. The composition according to any of claims 6 or 7, wherein the anti-angiogenesis agent is selected from the group consisting of an anti-VEGF antibody, an anti-VEGFR antibody and a tyrosine kinase inhibitor.

10. The composition according to any of claims 6 to 9, wherein the anti-VEGF antibody is bevacizumab, wherein the anti-VEGFR antibody is DC101 and/or wherein the tyrosine kinase inhibitor is sunitinib.

11. The composition according to any of claims 7 to 10 for use in the treatment of cancer, wherein said cancer is resistant to anti-angiogenic treatment.

12. The composition for use according to the preceding claim, wherein the cancer is renal cancer, breast cancer or another cancer intrinsically or acquired refractory to anti-angiogenic therapy.

13. An *in vitro* method for determining whether a tumor in patient is resistant or sensitive to an anti-angiogenesis agent that comprises
(i) determining the expression level of PD-ECGF in a sample from said patient, and
(ii) comparing the expression level in (i) to a reference value,
wherein an increased expression level of PD-ECGF with respect to said reference level is indicative that the tumor is resistant to anti-angiogenesis agent, and a decreased expression level of PD-ECGF with respect to said reference level is indicative that the tumor is sensitive to an anti-angiogenesis agent.

14. The method according to the preceding claim, wherein the anti-angiogenesis agent is selected from the group consisting of an anti-VEGF antibody, an anti-VEGFR antibody and a tyrosine kinase inhibitor.

15. The method according to claim 14 wherein the anti-VEGF antibody is bevacizumab, wherein the anti-VEGFR antibody is DC101 and/or wherein the tyrosine kinase inhibitor is sunitinib.
